# EUROPEAN PATENT APPLICATION

(11) **EP 4 480 470 A2**
(43) Date of publication of application: **25.12.2024**
(21) Application number: 24183679.0
(22) Date of filing: 21.06.2024
(51) Int. Cl.: A61J 3/07

(54) **SYSTEMS AND METHODS FOR LOADING CAPSULES WITH RADIOACTIVE MATERIALS FOR PHARMACEUTICAL, DIAGNOSTIC, AND/OR OTHER PURPOSES**

(30) Priority: 21.06.2023 US 202363509322 P; 23.06.2023 US 202363510065 P
(71) Applicant: EC2 Technologies, LLC, Phoenix, AZ 85020 (US); International Isotopes Inc., Idaho Falls, ID 83401 (US)
(72) Inventor: Zahn, Nathan Lihai, Phoenix (US); Candelaria, Leroy, Phoenix (US); Garza, Lucio, Phoenix (US); Laflin, Stephen, Idaho Falls (US); Wride, Richard, Idaho Falls (US)
(74) Representative: Forresters IP LLP

(57) **Abstract**

The present technology is directed to systems and methods for filing capsules with radioactive materials. In some embodiments, the present technology includes a capsule filling system that can transfer doses of a radioactive solution such as lodine-131 or another radioactive isotope from a source container to one or more capsules ready for medical or other use. The capsule filling systems can be operated remotely, and are therefore expected to reduce user exposure to radioactive materials during the capsule filling process. Further, the capsule filling systems can be partially or fully automated, which is further expected to reduce user exposure to radioactive materials and/or increase the efficiency of preparing capsules.

## Description

### CROSS-REFERENCE TO RELATED APPLICATIONS

The present application claims priority to U.S. Provisional Patent Application No. 63/509,322, filed June 21, 2023, and U.S. Provisional Patent Application No. 63/510,065, filed June 23, 2023, each of which is incorporated by reference herein in its entirety.

### TECHNICAL FIELD

The present disclosure is generally related to systems and associated methods for filling capsules with radioactive materials, such as to prepare radioactive capsules for pharmaceutical, diagnostic, and/or other purposes.

### BACKGROUND

Radioactive materials have long been used in healthcare settings, including in the diagnosis and treatment of various patient conditions. For example, lodine-131 is a radioactive isotope of iodine that is frequently used to treat various types of cancer. As a result, healthcare professionals at pharmacies, hospitals, and other healthcare settings could potentially be exposed to radiation, particularly when such professionals are involved with preparing radioactive doses to administer to a patient.

### BRIEF DESCRIPTION OF THE DRAWINGS

FIG. 1 illustrates an automated capsule filling system configured in accordance with embodiments of the present technology.
FIGS. 2A-2F illustrate additional features of a needle assembly, a vial transport assembly, and a moveable support assembly of the system of FIG. 1.
FIG. 3 illustrates additional features of the vial transport assembly of the system of FIG. 1.
FIGS. 4A and 4B illustrate additional features of a pig assembly of the system of FIG. 1.
FIGS. 5A-5C illustrate additional features of a vial holder assembly and a capsule capping assembly of the system of FIG. 1.
FIGS. 6A-6C illustrate additional features of a calibration assembly of the system of FIG. 1.
FIGS. 7A-7U illustrate various stages of an operation of using the system of FIG. 1 to prepare a radioactive capsule in accordance with embodiments of the present technology.
FIG. 8 illustrates a transport container for holding radioactive capsules and configured in accordance with embodiments of the present technology.

### DETAILED DESCRIPTION

The present technology is generally directed to systems and methods for filing capsules with materials, and in particular, radioactive materials. For example, in some embodiments the present technology includes a capsule filling system that can transfer doses of a radioactive solution, such as lodine-131 or another radioactive isotope, from a source container to one or more capsules ready for medical or other uses. As described in detail throughout this Detailed Description, embodiments of the capsule filling systems can be operated remotely, and are therefore expected to reduce user exposure to radioactive materials during the capsule filling process. Further, the capsule filling systems described herein can be partially or fully automated, which is further expected to reduce user exposure to radioactive materials and/or increase the efficiency of preparing capsules containing radioactive material.

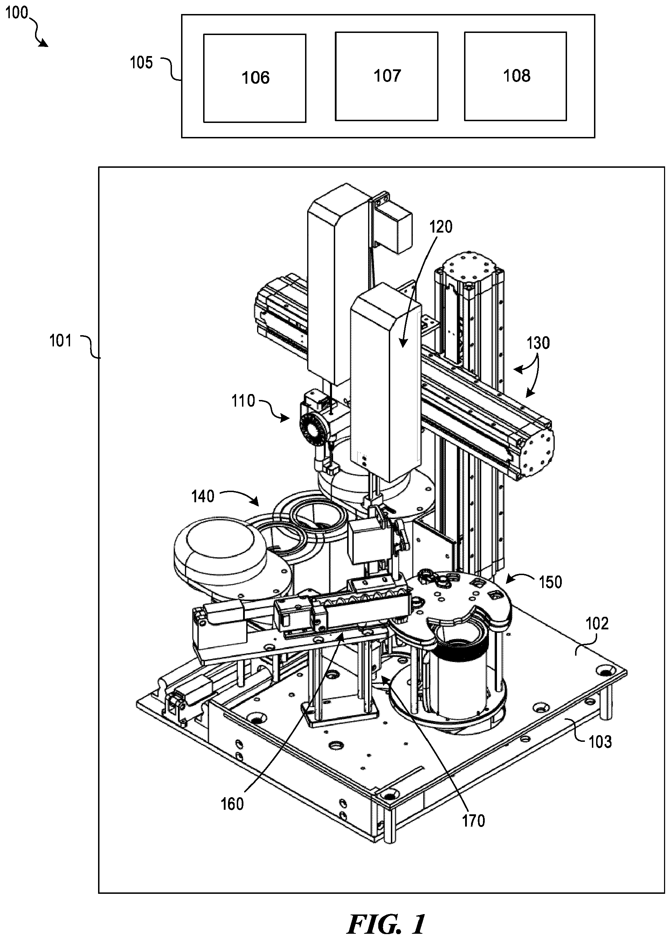

Certain details are set forth in the following description and in FIGS. 1-8 to provide a thorough understanding of various embodiments of the present technology. In other instances, well-known structures, materials, operations, and/or systems often associated with the handling of radioactive materials are not shown or described in detail in the following disclosure to avoid unnecessarily obscuring the description of the various embodiments of the technology. Those of ordinary skill in the art will recognize, however, that the present technology can be practiced without one or more of the details set forth herein, or with other structures, methods, components, and so forth.

The terminology used below is to be interpreted in its broadest reasonable manner, even though it is being used in conjunction with a detailed description of certain examples of embodiments of the technology. Indeed, certain terms may even be emphasized below; however, any terminology intended to be interpreted in any restricted manner will be overtly and specifically defined as such in this Detailed Description section.

The accompanying Figures depict embodiments of the present technology and are not intended to be limiting of its scope. The sizes of various depicted elements are not necessarily drawn to scale, and these various elements may be arbitrarily enlarged to improve legibility. Component details may be abstracted in the Figures to exclude details such as position of components and certain precise connections between such components when such details are unnecessary for a complete understanding of how to make and use the disclosed technology.

Many of the details, dimensions, angles, and other features shown in the Figures are merely illustrative of particular embodiments of the present technology. Accordingly, other embodiments can have other details, dimensions, angles, and features without departing from the spirit or scope of the present disclosure. In addition, those of ordinary skill in the art will appreciate that further embodiments of the present technology can be practiced without several of the details described below.

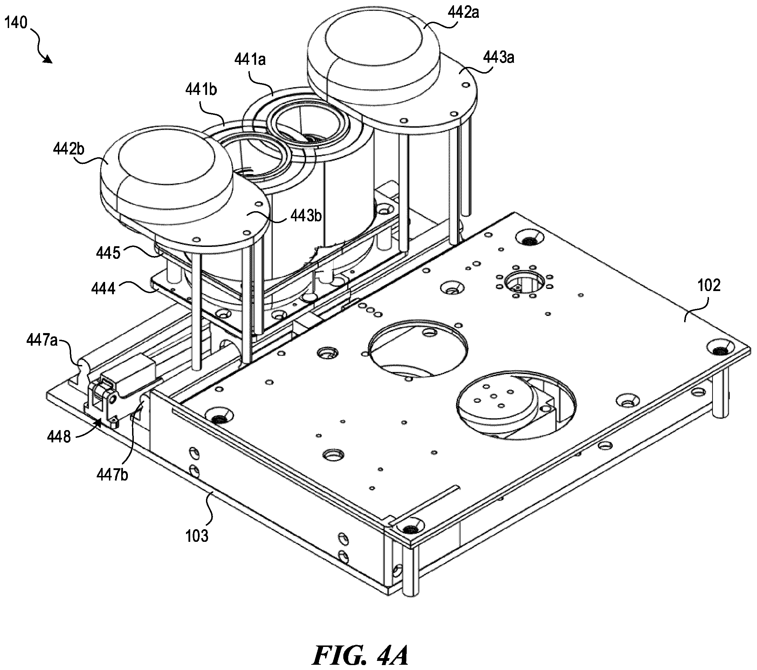

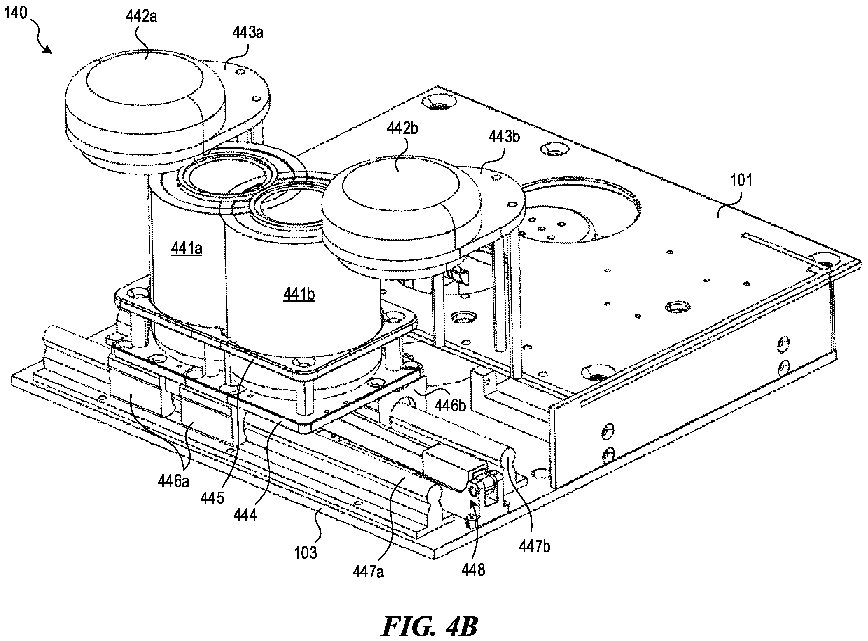

In the Figures, identical reference numbers identify identical, or at least generally similar, elements. To facilitate the discussion of any particular element, the most significant digit or digits of any reference number refers to the Figure in which that element is first introduced. For example, element 410 is first introduced and discussed with reference to Figure 4.

As used herein, the use of relative terminology, such as "about", "approximately", "substantially" and the like refer to the stated value plus or minus ten percent. For example, the use of the term "about 100" refers to a range of from 90 to 110, inclusive. In instances in which the context requires otherwise and/or relative terminology is used in reference to something that does not include a numerical value, the terms are given their ordinary meaning to one skilled in the art.

FIG. 1 illustrates an isometric view of an automated capsule filling system 100 ("the system 100") configured in accordance with embodiments of the present technology. As described in detail throughout this Detailed Description, the system 100 can automatically or semi-automatically transfer a dose of a radioactive agent, such as lodine-131 or another radioactive isotope, from a source container (e.g., motherload vial) into a capsule or other container ready for pharmacological or diagnostic use. The system 100 can be fully enclosed and designed to transfer radioactive agents from the source container to the capsule with minimal (e.g., no) direct user intervention. For example, to the extent a user must interact with the system 100 to prepare the radioactive capsules, the user can do so using a remote controller that can be spaced apart from (e.g., in a different room from) the portion of the system 100 handling radioactive material. In this way, the system 100 is expected to minimize or at least reduce radioactive exposure of pharmacists and other medical professional during the capsule filling process.

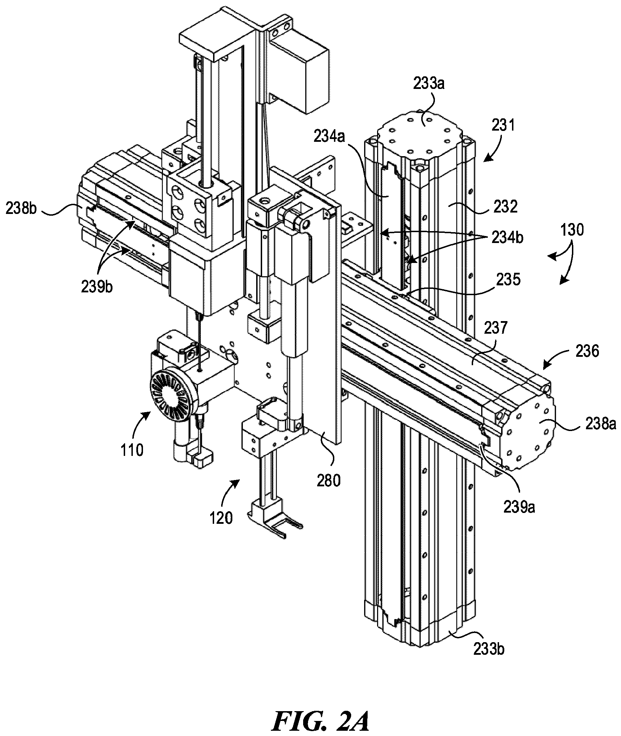

The system 100 includes a plurality of assemblies that operate together to transfer doses of the radioactive agent from a source vial to a capsule. For example, the system 100 includes a needle assembly 110 for transferring the radioactive agent from the source container to the capsule, a vial transport assembly 120 for transporting prepared capsules between various other assemblies, a moveable support assembly 130 for moving the needle assembly 110 and the vial transport assembly 120, a pig assembly 140 for holding the source container, a vial holder assembly 150 for holding capsules during the filling process, a capsule capping assembly 160 for enclosing the capsules once they have been filled with radioactive material, and a dose calibration assembly 170 for testing the radioactivity of prepared/filled capsules. Each of the foregoing assemblies is described in greater detail below with reference to FIGS. 2A-6C. The foregoing assemblies are supported on a first base plate 102 and/or a second base plate 103, as also described in greater detail below. The system 100 can further include a housing or outer structure 101 (shown schematically in Figure 1) that encloses each of the foregoing assemblies to provide a closed environment for capturing, retaining, and/or venting any escaped radioactive gases. Accordingly, in some embodiments, the housing 101 can be composed at least in part of a material that is impermeable or at least substantially impermeable to radiation. In some embodiments, the housing 101 can be composed of a transparent or semi-transparent material to permit a user to observe operation of the system 100.

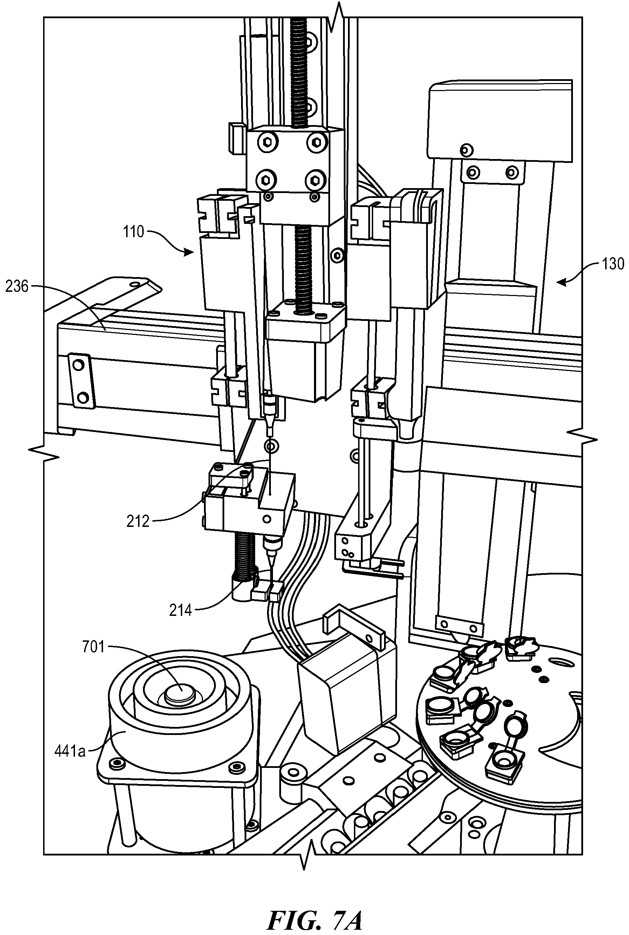

The system 100 can further include a computing system or controller 105 for controlling operation of the system 100. The controller 105 can be configured to control the system 100 via a wired and/or wireless connection(s). When the system 100 is in use, the controller 105 can be spaced apart from the housing 101 (e.g., placed in a different room than the housing 101) to reduce user exposure to radioactive materials within the housing 101. As shown, the controller 105 can include a memory 106 (e.g., non-transitory computer readable media) storing instructions executable by a processor 107 to control the operation of the system 100. For example, the operating system may control the system 100 in response to, e.g., human operator instructions, to perform any of the operations described throughout this Detailed Description, including the operations described below with reference to FIGS. 7A-7U. In some embodiments, the system 100 can further include a user interface 108 (e.g., a graphical user interface, a keypad, a touchscreen, a handheld remote, etc.) that enables a user to control various aspects of the system 100 (e.g., dose setting, initiating filling procedure, terminate filling procedure, vent radioactive materials, etc.).

FIG. 2A is an isometric view of the needle assembly 110, the vial transport assembly 120, and the moveable support assembly 130, with the other assemblies of the system 100 omitted for purposes of illustration. As shown, the needle assembly 110 and the vial transport assembly 120 can be coupled to a mounting or support plate 280, which in turn can be coupled to the support assembly 130. As described in detail below, the support assembly 130 can selectively move the needle assembly 110 and the vial transport assembly 120 (e.g., horizontally and/or vertically) by selectively moving the support plate 280.

The support assembly 130 includes a first arm 231 and a second arm 236. As shown, the first arm 231 can be oriented generally vertically and the second arm 236 can be oriented generally horizontally. The first arm 231 can be coupled to the first base plate 102 and/or the second base plate 103 (FIG. 1) of the system 100 via screws, bolts, brackets, and/or other suitable fasteners (not shown). The second arm 236 can be coupled to the first arm 231 via a support arm bracket 235. The support plate 280 can also be coupled to the second arm 236 via a similar bracket (not shown). As described below, the second arm 236 can be configured to move relative to the first arm 231 to control a vertical position (e.g., height) of the support plate 280. As also described below, the support plate 280 can be configured to move relative to the second arm 236 to control a horizontal position (e.g., lateral position) of the support plate 280.

The first arm 231 includes a first arm frame 232 and a first arm track or rail 234a, both of which extend generally vertically between a first end cap 233a and a second end cap 233b. The first arm frame 232 and the first arm rail 234a are sized and shaped to define two elongated, parallel slots 234b extending therebetween and oriented in a generally vertical direction. The two elongated slots 234b can slidably receive corresponding extensions of the bracket 235. The first arm 231 can further include a first linear actuator or motor (not shown) for engaging the extensions on the bracket 235 to selectively slide the bracket 235 "up and down" along the elongated slots 234b. In this way, the vertical position (e.g., height) of the bracket 235 can be selectively controlled by controlling a position of the bracket 235 within the elongated slots 234b. And because the bracket 235 is coupled to the second arm 236, the vertical position (e.g., height) of the second arm 236 can be selectively controlled by controlling a position of the bracket 235 within the elongated slots 234b.

Similar to the first arm 231, the second arm 236 includes a second arm frame 237 and a second arm track or rail 239a, both of which extend generally horizontally between a first end cap 238a and a second end cap 238b. The second arm frame 237 and the second arm rail 239a are sized and shaped to define two elongated, parallel slots 239b extending therebetween and oriented in a generally horizontal direction. The two elongated slots 239b can slidably receive two corresponding extensions (not shown) extending from a back surface of the support plate 280. The second arm 236 can further include a second linear actuator or motor (not shown) for engaging the extensions on the support plate 280 that are positioned within the elongated slots 239b to selectively slide the support plate 280 "side to side" along the elongated slots 239b. Accordingly, the horizontal (e.g., lateral) position of the support plate 280 can be selectively controlled by sliding the support plate 280 along the elongated slots 239b.

As set forth above, in the illustrated embodiment the support plate 280 carries the needle assembly 110 and the vial transport assembly 120. As further set forth above, the support assembly 130 can translate the support plate 280 vertically (e.g., in the y-direction) and horizontally (e.g., in the x-direction). Accordingly, the support assembly 130 can selectively translate the needle assembly 110 and the vial transport assembly 120 in both the y-direction and the x-direction (e.g., providing two degrees of freedom of translation).

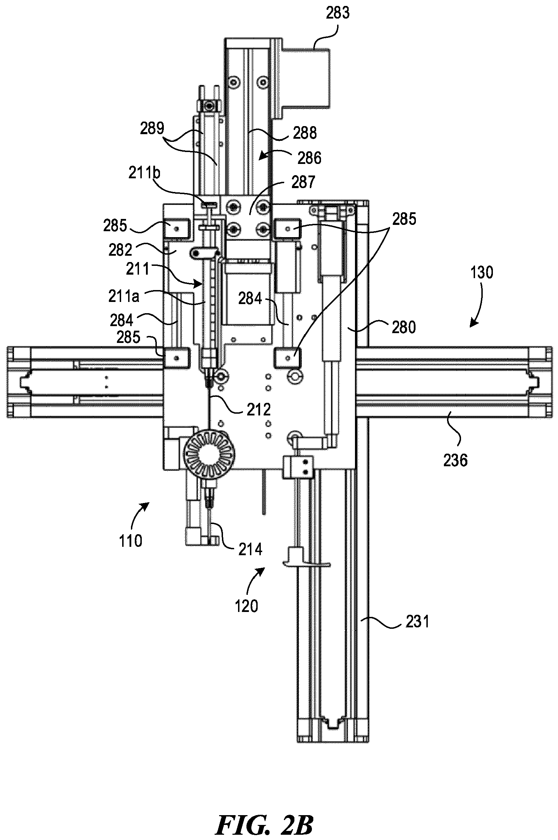

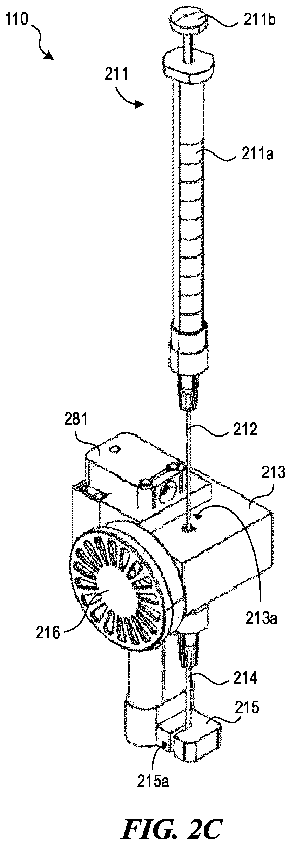

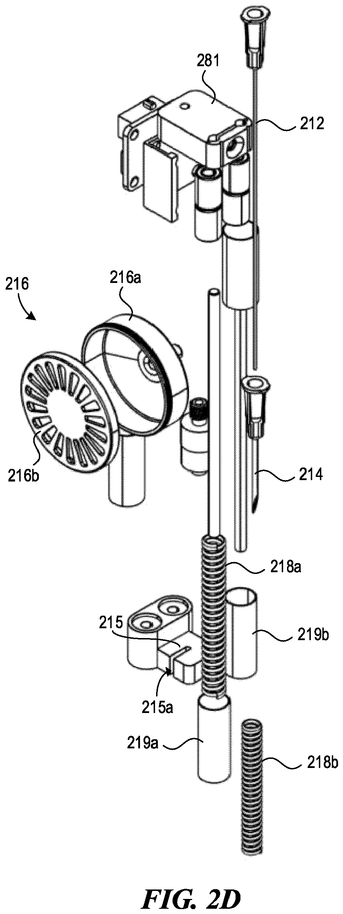

FIG. 2B is a front view of the needle assembly 110, the vial transport assembly 120, and the support assembly 130. In the illustrated embodiment, the needle assembly 110 includes a syringe 211 coupled to a syringe support frame 282. As described in greater detail with reference to FIGS. 2C and 2D, the syringe 211 includes a syringe barrel 211a, a syringe plunger 211b, and a first needle 212. The first needle 212 is sized and shaped to extend coaxially within a central passage of a second needle 214 of the needle assembly 110. In use, withdrawing the syringe plunger 211b from the syringe barrel 211a reduces the pressure within the first needle 212 and creates a suction force. Thus, if the first needle 212 is submerged in solution as the syringe plunger 211b is withdrawn upwardly, the suction force draws the solution into the first needle 212. Similarly, compressing the syringe plunger 211b relative to the syringe barrel 211a increases the pressure within the first needle 212 and causes solution within the first needle 212 to be expelled therefrom.

The syringe support frame 282 (and thus the syringe 211) can translate relative to the support plate 280 to further control a vertical position (e.g., height) of the syringe 211 and the first needle 212. For example, the syringe support frame 282 can be slidably coupled to two vertically extending shafts 284, which can be coupled to the support plate 280 via corresponding shaft mounts 285. A support frame motor 283 can control a position of the syringe support frame 282 along the shafts 284 between the shaft mounts 285 (e.g., by sliding the syringe support frame 282 up and/or down along the shafts 284). In this way, the motor 283 can control the vertical position (e.g., height) of the syringe 211 and the first needle 212 relative to the support plate 280.

Of note, moving the syringe support frame 282 along the shafts 284 does not move the syringe plunger 211b relative to the syringe barrel 211a, and thus does not induce a suction or expulsion force within the first needle 212. Instead, the needle assembly 110 further includes a syringe actuator 286 that can move vertically to selectively move the syringe plunger 211b relative to the syringe barrel 211a to draw fluid into the first needle 212 and subsequently expel the fluid from the first needle 212. The syringe actuator 286 can include a sliding component 287 configured to translate relative to the syringe support frame 282 along a syringe actuator shaft 288 and a plurality of auxiliary shafts 289. The sliding component 287 can be coupled to the syringe plunger 211b (e.g., via a male-female connection or other suitable connection) such that as the sliding component 287 slides along the actuator shaft 288 and the auxiliary shafts 289, the syringe plunger 211b moves with it. Because the syringe barrel 211a is translationally locked relative to the syringe support frame 282, the syringe barrel 211a does not translate with the syringe plunger 211b. Instead, the syringe plunger 211b moves relative to the syringe barrel 211a as the sliding component 287 moves along the actuator shaft 288 and the auxiliary shafts 289. The syringe actuator 286 can include a linear actuator or other suitable motor (not shown) for selecting moving the sliding component 287 along the actuator shaft 288 and auxiliary shafts 289.

FIG. 2C is an enlarged view of certain features of the needle assembly 110 including the syringe 211, with other features of the needle assembly 110 omitted for clarity. As shown, the needle assembly 110 includes a needle housing 213 having a needle passage 213a extending therethrough for receiving the first needle 212. The needle housing 213 can be coupled to (e.g., translationally locked relative to) the support plate 280 (FIGS. 2A and 2B) using screws, pins, or other fasteners. Accordingly, the first needle 212 can move relative to the needle housing 213 by operating the support frame motor 283 (FIG. 2D) to translate the syringe support frame 282 relative to the support plate 280, as described above with respect to FIG. 2B. The needle housing 213 can further include a needle sensor 281 (e.g., an optical sensor) configured to track a position of the first needle 212 (e.g., relative to the housing 213). The needle sensor 281 can provide positional information about the first needle 212 to verify contact between the first needle 212 and a source container, verify a dose of solution withdrawn from a source container, and/or verify contact between the first needle 212 and a capsule for receiving the dose of solution. In some embodiments, the system 100 can control movement of the needle assembly 110 based on feedback from the needle sensor 281.

As set forth above, the needle assembly 110 includes the second needle 214. The second needle 214 extends between the needle housing 213 and a needle guide 215. The needle guide 215 includes a slot 215a extending between an upper surface and a lower surface of the needle guide 215 and sized and shaped to receiving a distal tip portion of the second needle 214. The needle guide 215 can also be sized and shaped to align with, and be positioned in apposition to, a source container (not shown) holding, e.g., a radioactive solution. In operation, the needle guide 215 can be pushed toward the needle housing 213 such that the distal end of the second needle 214 advances through the slot 215a in the needle guide 215 and into the source container. As described in detail below, the first needle 212 can then be moved concentrically through the second needle 214 to withdraw a dose of the solution from the source container. That is, the first needle 212 extends coaxially within the second needle 214, forming an annular channel between the first needle 212 and the second needle 214. The needle assembly 110 further includes a filter 216 for capturing radioactive material (e.g., off-gas, vapor, or solution) escaping through the annular channel formed between the first needle 212 and the second needle 214.

As best shown in FIG. 2D, which is an exploded view of the features shown in FIG. 2C (with the needle housing 213 omitted relative to FIG. 2C), the first needle 212 can have an outer diameter that is smaller than an inner diameter of the passage extending through the second needle 214. This enables the first needle 212 to extend through (e.g., be co-axial with) the second needle 214, as described above. For example, in some embodiments the first needle 212 has an outer diameter that is between about 5% and about 95% less than the inner diameter of the passage through the second needle 214. In some embodiments, a ratio of the inner diameter of the second needle 214 to the outer diameter of the first needle 212 can be between about 5:1 to about 1.2:1, or between about 3:1 to 1.5:1, orabout2:1.

As also shown in FIG. 2D, the needle assembly 110 can include a first biasing member (e.g., a first compression spring 218a) positioned within one or more first spring sleeves 219a and a second biasing member (e.g., a second compression spring 218b) positioned within one or more second spring sleeves 219b (collectively referred to as "the compression springs 218" and the "spring sleeves 219," respectively). When assembled, the compression springs 218 and the spring sleeves 219 extend at least partially between the needle housing 213 (FIG. 2C) and the needle guide 215. In operation, the springs 218 are at least partially compressed in response to the needle guide 215 being pushed down upon another structure (e.g., a source container; not shown). As the springs 218 compress, a distance between the needle housing 213 and the needle guide 215 decreases. However, because the second needle 214 is slidably received within the slot 215a of the needle guide 215, the second needle 214 does not move with the needle guide 215. As a result, a distal end of the second needle 214 extends past the needle guide 215 and can puncture or otherwise enter the source container positioned beneath the needle guide 215.

As also best shown in FIG. 2D, the filter 216 includes a filter base 216a and a filter cover 216b having a plurality of vent apertures. As set forth above, the filter 216 can be configured to capture any radioactive fumes or particles that escape the source container within the annular channel formed between the first needle 212 and the second needle 214. For example, the filter base 216a can be configured to hold a radioactive absorbing material (e.g., charcoal, a charcoal containing mixture, an active charcoal mesh, or other suitable materials known in the art; not shown) that captures and retains radioactive materials. The filter cover 216b can help retain the radioactive absorbing material and any captured radioactive material within the filter base 216a.

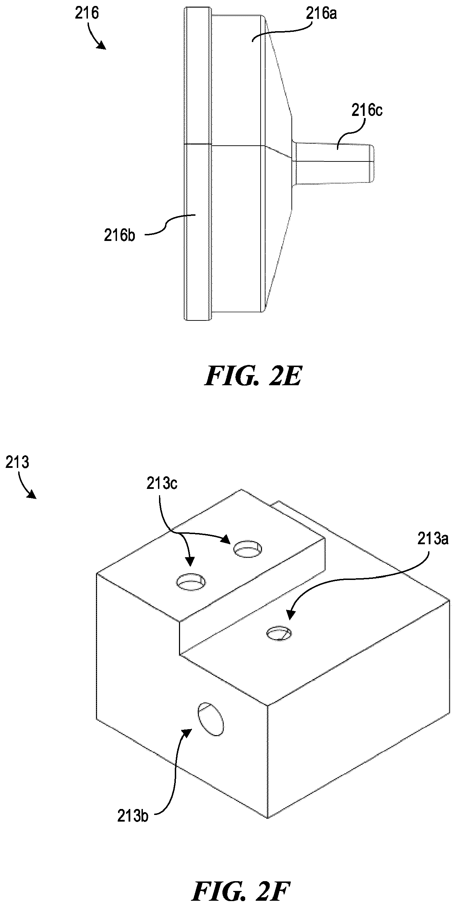

FIG. 2E is a side view of the filter 216. In addition to the filter base 216a and the filter cover 216b, the filter 216 can further include a filter nipple or other coupling element 216c. As shown, the filter nipple 216c projects outwardly from the filter base 216a on a side generally opposite to the filter cover 216b. The filter nipple 216c can have an internal lumen (not shown) fluidly coupled to an interior of the filter base 216a. That is, gases can pass through the filter nipple 216c to enter the interior of the filter base 216b. The filter nipple 216c can further be sized and shaped to be releasably coupled to a corresponding filter receiving feature on the needle housing 213 (FIG. 2C), as described below with reference to FIG. 2F. In this way, a user can periodically replace the filter 216 by disengaging the filter 216 from the corresponding filter receiving feature in the needle housing 213. In some embodiments, the filter nipple 216c can be composed at least in part of a partially flexible and/or elastic material that conforms to the filter receiving feature to provide a hermetic, or substantially hermetic, seal therebetween.

FIG. 2F is an isometric view of the needle housing 213. In addition to the needle passage 213a for receiving the first needle 212 (FIGS. 2B-2D), the needle housing 213 can include a second lumen or passage 213b that can operate as the filter receiving feature. That is, the filter 216 (FIG. 2E) can be releasably coupled to the needle housing 213 by inserting the filter nipple 216c into the second passage 213b. Of note, when the filter nipple 216c is inserted into the second passage 213b, the interior of the filter nipple 216c, and thus the interior of the filter base 216a, is in fluid communication with the second passage 213b. Moreover, the second passage 213b can be fluidly coupled to the needle passage 213a (e.g., at a T-shaped or other suitable junction) within an interior of the needle housing 213. Thus, radioactive fumes escaping external to the first needle 212 but within the needle passage 213a can be routed into the filter 216 via the second passage 213b. In some embodiments, the second passage 213b can be lined with, or formed of, a material configured to capture and retain radioactive materials. For example, the second passage 213b may be coated with a sticky substance or resin such as a silica-based ion-exchange resin. Without intending to be bound by theory, incorporating a material that captures and retains radioactive materials into the second passage 213b is expected to further reduce the risk of radioactive fumes escaping from the needle assembly 110.

The needle housing 213 can also have additional features. For example, in the illustrated embodiment the needle housing 213 has a plurality (e.g., two) retention ports 213c. The retention ports 231c can be sized and shaped to receive corresponding pins on the needle sensor 281 (FIG. 2D) to couple the needle sensor 281 to the needle housing 213. In some embodiments, the retention ports 231 can be positioned on the needle housing 213 to ensure that the needle sensor 281 is properly aligned with the first needle 212 so that the needle sensor 281 can properly evaluate the first needle 212, as described above with reference to FIG. 2D.

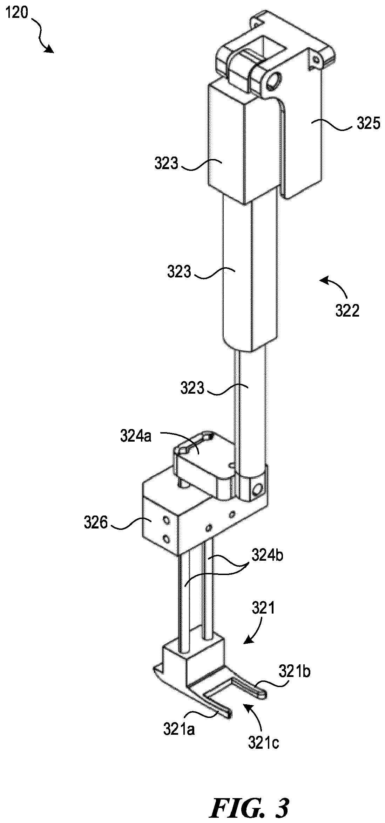

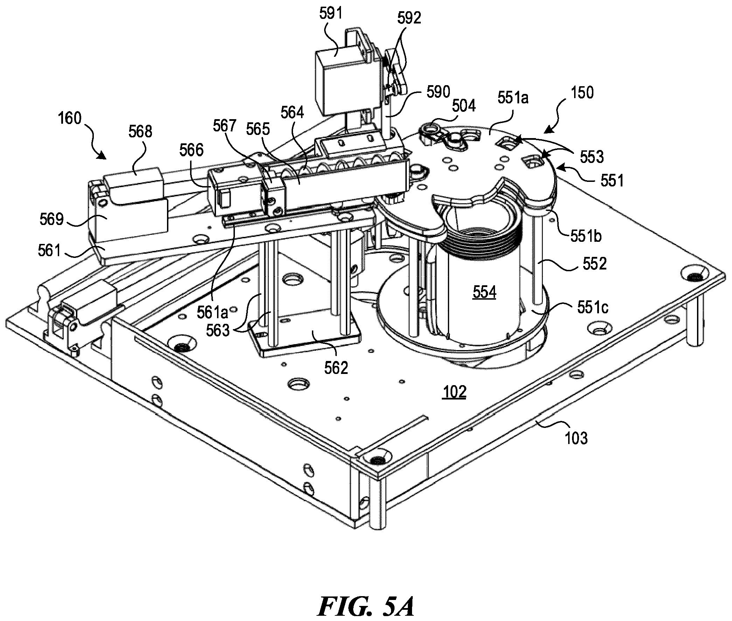

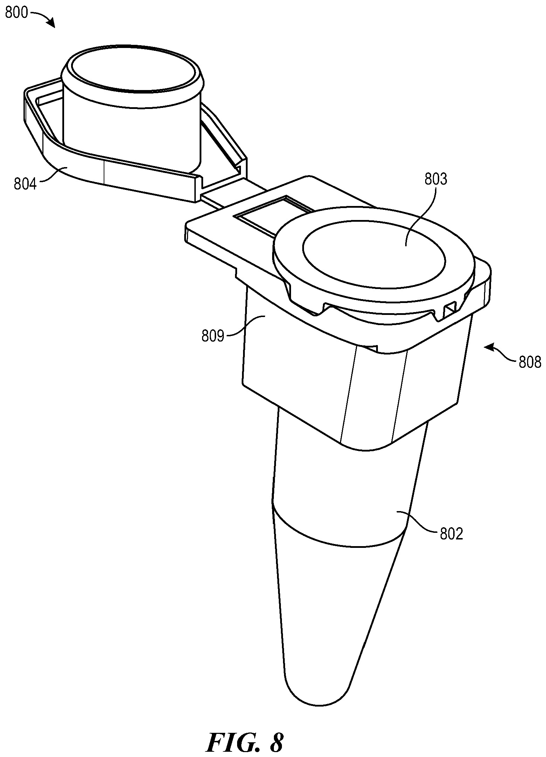

FIG. 3 is an isometric view of the vial transport assembly 120 of FIG. 1, with the other assemblies of the system 100 omitted for purposes of illustration. The vial transport assembly 120 includes a gripper or hand 321 configured to releasably grasp a vial or other transport container configured to hold a capsule, such as the transport container 504 described below with reference to FIG. 5A and/or the transport container 800 described below with reference to FIG. 8. For example, the gripper 321 can include a first prong, tine, or finger 321a and a second prong, tine, or finger 321b. The first prong 321a can be spaced apart from the second prong 321b to define a recess or gap 321c therebetween. The gap 321c can be sized and shaped to receive the transport container. Accordingly, although shown as having a generally rectangular shape, the gap 321c can have other suitable shapes, including crescent shaped, triangular, irregular, etc., depending on the size and shape of the transport container.

The vial transport assembly 120 can be coupled to the support plate 280 (FIGS. 1-2B) via a bracket 325 (e.g., a clevis bracket) and a guide 326. In some embodiments, the bracket 325 and guide 326 are translationally and rotationally locked to the support plate 280 (e.g., via screws, nuts, bolts, adhesives, and/or other fasteners). The position of the vial transport assembly 120 can therefore be adjusted in the y-direction and the x-direction by adjusting a position of the support plate 280 via actuation of the support assembly 130, described in detail above with reference to FIG. 2A.

The vial transport assembly 120 can further include a gripper actuator 322 that can move the gripper 321 relative to the support plate 280 (FIGS. 1-2B) to provide further positional control of the gripper 321. For example, the gripper actuator 322 can include a plurality of linearly nesting components 323 that sleeve together and extend between the bracket 325 and a coupling component 324a, which is coupled to the gripper 321 via one or more shafts 324b that are slidably retained within passages extending between upper and lower surfaces of the guide 326.

An actuator motor (e.g., an electric motor; not shown) can control the telescoping movement between the nesting components 323, and thus control a combined axial length of the nesting components 323. The gripper actuator 322 is shown in FIG. 3 in a fully extended state (i.e., a state with the least amount of nesting between the nesting components 323). To move the gripper 321 upwardly (e.g., toward the guide 326), the gripper actuator 322 can reduce the combined axial length of the nesting components 323 by increasing the nesting of the nesting components 323. This pulls the coupling component 324a, the shafts 324b, and the gripper 321 upwardly, with the gripper 321 moving toward the guide 326 and the slotted frame 325. To move the gripper 321 downwardly (e.g., away from the guide 326), the gripper actuator 322 can increase the combined axial length of the nesting components 323 by decreasing the nesting of the nesting components 323. This pushes the coupling component 324a, the shafts 324b, and the gripper 321 downward, with the gripper moving away from the guide 326 and the slotted frame 325. Accordingly, the gripper actuator 322 is configured to provide additional positional control of the gripper 321 in the y-direction.

FIG. 4A is a front isometric view of the pig assembly 140, the first base plate 102, and the second base plate 103, with the other assemblies of the system 100 omitted for purposes of illustration. FIG. 4B is a side isometric view of the same features of FIG. 4A. Referring collectively to FIGS. 4A and 4B, the pig assembly 140 can include a first pig 441a and a second pig 441b (collectively referred to as "the pigs 441"). (As is known, the term "pig" is commonly used to describe a container used to ship or store radioactive materials.) The pigs 441 can be configured to house and retain a radioactive source material, such as a source canister or container holding a radioactive solution. For example, the pigs 441 are shown as generally cylindrical elements with an interior chamber for holding the source container, although other configurations are possible and within the scope of the present technology. The pigs 441 can therefore also be composed at least in part of a radioactive shielding material such as lead.

The pig assembly 140 can further include a first pig cap 442a and a second pig cap 442b (collectively referred to as "the pig caps 442") for enclosing the first pig 441a and the second pig 441b, respectively. The first pig cap 442a and the second pig cap 442b can be positioned on a first pig cap support plate 443a and a second pig cap support plate 443b, respectively, when the system 100 is being actively used (e.g., to transfer doses of radioactive solution from the source containers within the pigs 441 to one or more capsules). When the system 100 is not being actively used, the pig caps 442 can be positioned over the pigs 441 to further reduce or minimize radioactive venting from the source containers positioned within the pigs.

The pigs 441 can be positioned on a pig support plate 444. The pig assembly 140 can further include one or more pig positioning plates 445, which can have openings or slots that guide the pigs 441 into predetermined positions on the support plate 444. In the illustrated embodiment, for example, the positioning plate 445 includes two openings sized and shaped to receive the corresponding first pig 441a and the second pig 441b. The positioning plate 445 therefore ensures the pigs 441 are positioned in precise and known positions relative to the support plate 444 and other components of the system 100, which is expected to be useful when the system 100 selectively moves one of the pigs 441 to align with the needle assembly 110, as described below. The support plate 444 can be coupled to, but at least partially spaced apart from, the positioning plate 445 (e.g., by one or more spacers).

The pigs 441 can be translated relative to the first base plate 102 and/or the second base plate 103 via a rail system to control a position of the pigs 441. For example, the pig assembly 140 can include a first rail 447a and a second rail 447b (collectively referred to as "the rails 447"). The pig support plate 444 can be slidably coupled to the first rail 447a via one or more first linear sliders 446a and slidably coupled to the second rail 447b via one or more second linear sliders 446b (collectively referred to as "the linear sliders 446"). The pig assembly 140 further includes a linear actuator 448 (which can also be referred to herein as "the pig actuator 448") operatively coupled to the support plate 444 such that it can move the support plate 444 (and thus the pigs 441) by sliding the linear sliders 446 along the rails 447.

In operation, the pig actuator 448 enables the system 100 to selectively align either the first pig 441a or the second pig 441b with the needle assembly 110 (FIGS. 2A-2D). Thus, in embodiments in which the first pig 441a and the second pig 4441b contain different radioactive materials (or the same radioactive material at different stages of decay), the system 100 can selectively control which pig 441 is aligned with the needle assembly 110, and thus which radioactive material is being transferred to capsules using the needle assembly 110. In this way, the system 100 can provide more options for filing capsules compared to filling systems having a single, immovable pig. This is also expected to reduce user exposure to radioactive materials because it may reduce the frequency with which source containers must be replaced. Although the pig assembly 140 is shown as having two pigs 441, in some embodiments the pig assembly 140 can have more pigs 441, such as three, four, five, six, seven, or more. In other embodiments, the pig assembly 140 may have a single pig 441.

FIG. 5A is an isometric view of the vial holder assembly 150, the capsule capping assembly 160, the first base plate 102, and the second base plate 103, with the other assemblies of the system 100 omitted for purposes of illustration. The vial holder assembly 150 is used to hold one or more transport containers or vials 504 (such as those described below with reference to FIG. 8) that are configured to hold and transport loaded/filled capsules. In the illustrated embodiment, the vial holder assembly 150 includes a rotatable carousel 551 having a first plate 551a, a second plate 551b, and a third plate 551c. The first plate 551a can have a plurality of openings or slots 553 for receiving and releasably retaining the transport containers 504. In some embodiments, the slots 553 are sized and shaped to ensure that the transport containers 504 remain in a predetermined orientation (e.g., upright) when the transport containers 504 are positioned within the slots 553. This ensures that the system 100 can transfer a dose of radioactive solution into capsules (e.g., load or fill the capsules) contained within the transport containers 504 (e.g., via the needle assembly 110 shown in FIG. 2A-2D) and cap the capsules using the capsule capping assembly 160 (described below).

As shown, the first plate 551a and the second plate 551b are positioned generally proximate each other, while the third plate 551c is spaced apart from the second plate 551b by a plurality of elongated spacers 552. A capsule receptacle 554 can be positioned within the space formed between the second plate 551b and the third plate 551c. As described in greater detail below, transport containers 504 holding filled/loaded capsules can be delivered to the capsule receptacle 554 (e.g., using the vial transport assembly 120 shown in FIG. 3) for storage until subsequent user retrieval.

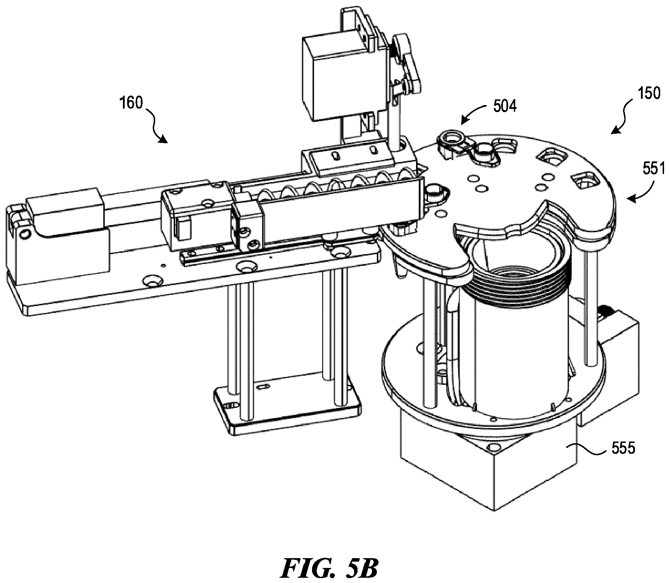

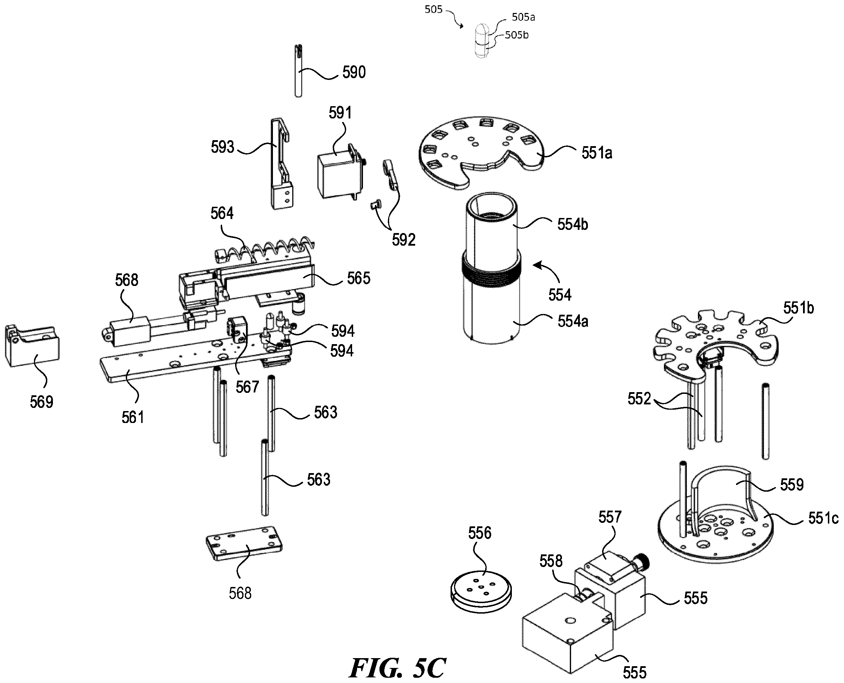

As best shown in FIG. 5B, which illustrates the vial holder assembly 150 and the capsule capping assembly 160 with the first base plate 102 and the second base plate 103 omitted, the rotatable carousel 551 sits on a turntable support 555. As described below with reference to FIG. 5C, the rotatable carousel 551 can rotate relative to the turntable support 555 to change which slot 553 (and thus which transport container 504) is aligned with the capsule capping assembly 160.

FIG. 5C is an exploded view of the features shown in FIG. 5B. The vial holder assembly 150 can include a rotatable turntable base 556 rotationally pinned to the carousel 551 (e.g., rotatably locked to the third plate 551c using screws, pins, or other suitable fasteners). The vial holder assembly 150 can further include a carousel motor 557 that can rotate a rotatable shaft 558, which in turn can rotate the rotatable turntable base 556 relative to the turntable support 555. Because the rotatable turntable base 556 is rotationally pinned to the carousel 551, rotating the turntable base 556 causes corresponding rotation of the carousel 551. In this manner, the carousel motor 557 can selectively rotate the carousel 551 to change which transport container 504 is aligned with the capsule capping assembly 160. In some embodiments, the carousel motor 557 can rotate the carrousel 551 in both a clockwise and counterclockwise direction.

FIG. 5C also illustrates additional features of the capsule receptacle 554. In particular, the capsule receptacle 554 can include an inner sleeve 554b sized and shaped to sit within (e.g., be co-axial with) an outer sleeve 554a. The inner sleeve 554b and/or the outer sleeve 554a can be composed of a radiation blocking or shielding material (e.g., lead) to reduce the radiation emanating from capsules positioned within the receptacle 554. As shown, the vial holder assembly 150 can also include a positioner 559 (e.g., a curved plate) extending from the third plate 551c of the carousel 551. The positioner 559 can have a shape corresponding to the capsule receptacle 554 to ensure the capsule receptacle 554 is positioned correctly, e.g., to reduce the risk of "missing" the receptacle 554 when transporting loaded capsules thereto.

In some embodiments, the vial holder assembly 150 can have other configurations for holding and moving the transport containers 504. For example, the vial holder assembly 150 could include a conveyer belt having slots or other features for holding the transport containers 504, e.g., in addition to or in lieu of the carousel 551. In such embodiments, the vial holder assembly 150 can otherwise function similarly to the embodiment described with reference to FIGS. 5A-5C.

Returning to FIGS. 5A and 5C, the capsule capping assembly 160 "caps" capsules 505 (FIG. 5C0 retained within the transport containers 504 after the capsules have been loaded with radioactive material. Capsules can have a first (e.g., upper) half, portion, or shell 505a and a second (e.g., lower) half, portion, or shell 505b. When used with the system 100, the lower half 505b can be positioned within a transport container 504 and loaded with radioactive material. Once the lower half 505b of the capsule 505 is loaded with radioactive material, the capsule capping assembly 160 automatically connects the upper half of the capsule 505a to the lower half 505b to form the complete capsule 505. In some embodiments, the capsules 505 may have a dual-capsule or a "capsule-inside-a-capsule" arrangement, in which each capsule includes two first (e.g., upper) halves or shells 505a and two second (e.g., lower) halves or shells 505b. In such embodiments, the full inner capsule and the lower half of the outer capsule can be prepositioned within the transport container 504 before radioactive material is loaded into the inner capsule. The upper half of the outer capsule can then be connected to the lower half of the outer capsule using the capsule capping assembly 160.

Referring collectively to FIGS. 5A and 5C, the capsule capping assembly 160 includes a capsule capping assembly base plate 561 ("the base plate 561") that carries various features of the capsule capping assembly 160, and a capsule capping assembly mounting plate 562 ("the mounting plate 562") that securely couples the capsule capping assembly 160 to the first base plate 102. The base plate 561 can be spaced apart from the mounting plate 562 via one or more spacers 563. The spacers 563 generally have a height that corresponds to a height of the carousel 551 to ensure that the capsule capping assembly 160 has sufficient height to cap the capsules carried by the carousel 551.

The capsule capping assembly 160 further includes a shell delivery mechanism, which in the illustrated embodiment includes a spiral pusher 564, a spiral pusher frame 565, and a spiral pusher motor 566 that rotates the spiral pusher 564 within the spiral pusher frame 565. The spiral pusher 564 (which can also be referred to as an auger, a helical feed screw, etc.) can have a helical thread configured to carry the first (e.g., upper) halves of capsule shells from a capsule dispenser 567 to the carousel 551. For example, a capsule upper half can sit between adjacent helices of the spiral pusher 564. As the spiral pusher 564 rotates about its central, longitudinal axis, the capsule upper half moves distally until it reaches an end of the spiral pusher 564. Because the end of the spiral pusher 564 is aligned with the carousel 551, the capsule upper half falls into a transport container 504 carried by the container 551 and over a corresponding capsule lower half positioned within the transport container 504. In other embodiments, the capsule capping assembly 160 can include other shell delivery mechanisms, such as a conveyer belt for delivering the capsule upper halves to the carrousel 551.

The spiral pusher 564, the spiral pusher frame 565, the spiral pusher motor 566, and the capsule dispenser 567 can be coupled to the base plate 561 via a rail bracket 561a, which itself can be coupled to the base plate 561 via one or more connector pins 594 (FIG. 5C). In some embodiments, the capsule capping assembly 160 can include a linear actuator 568 that can move the spiral pusher frame 565, the spiral pusher 564, the spiral pusher motor 566, and the capsule dispenser 567 along the rail bracket 561a. In this way, the linear actuator 568 can control a relative position of the distal end of the spiral pusher 564, e.g., to ensure it aligns with a target transport container 504. The linear actuator 568 can be coupled to the base plate 561 via an actuator mount 569 or other suitable mechanism.

The capsule capping assembly 160 also includes a mechanism for pushing the capsule upper half over the capsule lower half after the capsule upper half has been delivered to the transport container 504 via the spiral pusher 564. For example, the capsule capping assembly 160 includes a push rod motor 591 that controls a push rod 590 via one or more articulating arms 592. In operation, the push rod motor 591 can control the articulating arms 592 to depress the push rod 590 and provide a downward force on a capsule upper half positioned within the transport container 504, e.g., to frictionally couple the capsule upper half to the capsule lower half. The push rod motor 591, the push rod 590, and the articulating arms 592 can be coupled to the base plate 561 via a motor mounting frame 593 (FIG. 5C).

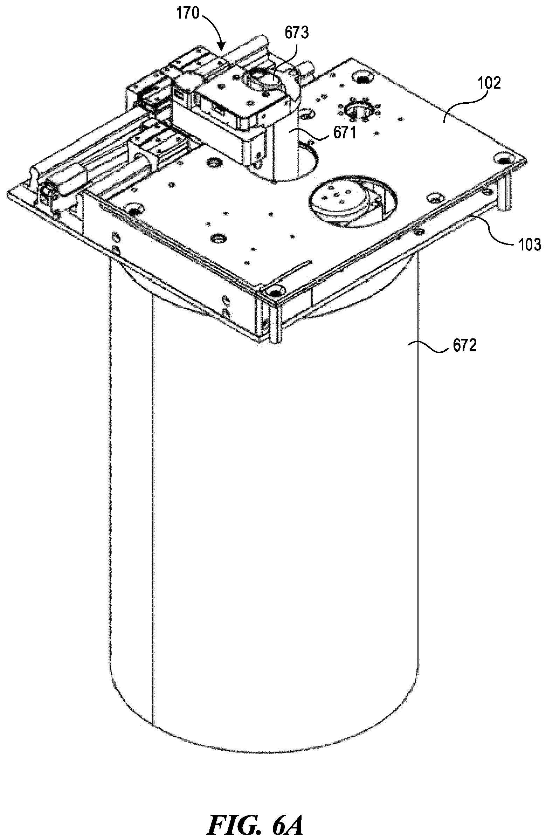

FIG. 6A is an isometric view of the dose calibration assembly 170, the first base plate 102, and the second base plate 103, with the other assemblies of the system 100 omitted for purposes of illustration. The dose calibration assembly 170 can be used to measure the radioactivity of capsules filled using the system 100. As shown, the dose calibration assembly 170 includes a dose calibrator well 671 (also referred to herein as "the well 671") and a dose calibrator basket 673 (also referred to herein as "the basket 673"). The dose calibration assembly 170 further includes a dose calibrator housing 672 positioned beneath the second base plate 103 that is configured to contain the calibration instruments (not shown). As described in greater detail below, a transport container 504 (FIG. 5A) carrying a loaded/filled capsule can be positioned in the dose calibrator basket 673, which can be lowered through the dose calibrator well 671 for calibration within the housing 672.

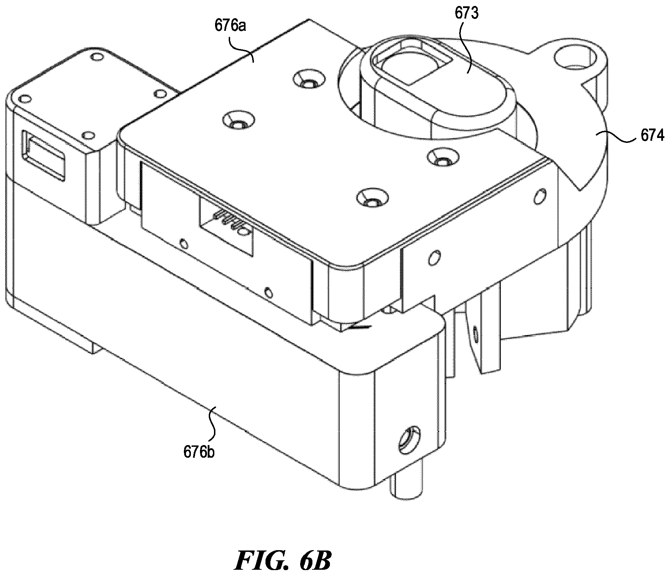

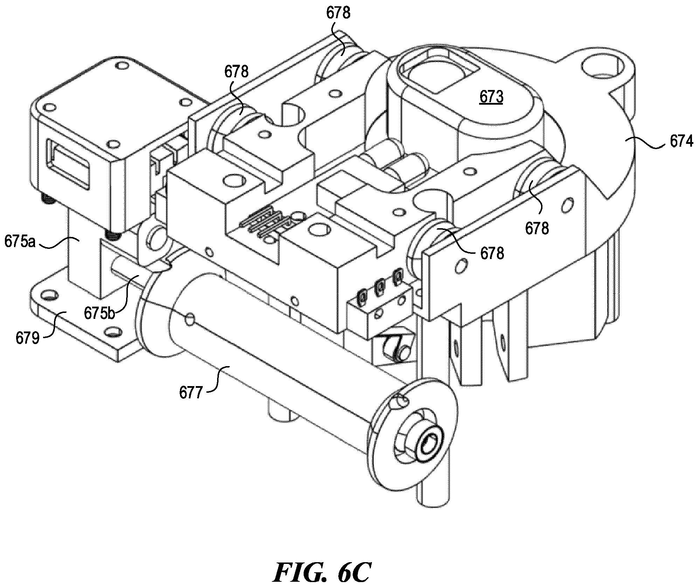

FIG. 6B is an enlarged view of certain features of the dose calibration assembly 170. For example, FIG. 6B illustrates the basket 673 positioned within a collar 674, which itself can sit above the well 671 (FIG. 6A). The dose calibration assembly 170 can further include a cover 676a covering the collar 674, and an outer housing 676b. FIG. 6C is the same view as shown in FIG. 6B, but with the cover 676a and the outer housing 676b removed to better illustrate additional features of the dose calibration assembly 170. As shown in FIG. 6C, the dose calibration assembly 670 includes a motor 675a coupled to the first base plate 102 (FIG. 6A) via a dose calibration assembly base plate 679. When activated, the motor 675a rotates a rotatable shaft 675b, which is rotationally keyed to, and thus rotates, a wrench wheel 677. One or more cables (not shown) can be wound around the wrench wheel 677, extend over a plurality of wheels 678 positioned within or proximate the collar 674, and couple to the basket 673. In operation, the motor 675a can rotate the wrench wheel 677 in a first direction (e.g., clockwise) to unwind/unspool the cable from the wrench wheel 677, increasing a length of cable between the wrench wheel 677 and the basket 673. This lowers the basket 673 into the well 671 and housing 672 (FIG. 6A). The motor 675a can also rotate the wrench wheel 677 in a second direction opposite the first direction (e.g., counterclockwise) to wind/spool the cable onto the wrench wheel 677, decreasing a length of cable between the wrench wheel 677 and the basket 673. This raises the basket 673, e.g., moving it up the well 671 and out of the housing 672. Accordingly, the system 100 can control the motor 675a to selectively lower and raise the basket 673 into the well 671 and housing 672 for dose calibration.

FIGS. 7A-7U illustrate a representative workflow for using the system 100 for loading a dose of radioactive material into a capsule. For ease of description and purposes of illustration, some of the images shown in FIGS. 7A-7U may not include every feature described above with reference to FIGS. 1-6B. However, one skilled in the art will readily appreciate how such omitted feature fits within the operation described for FIGS. 7A-7U based on the corresponding description of such features for FIGS. 1-6B.

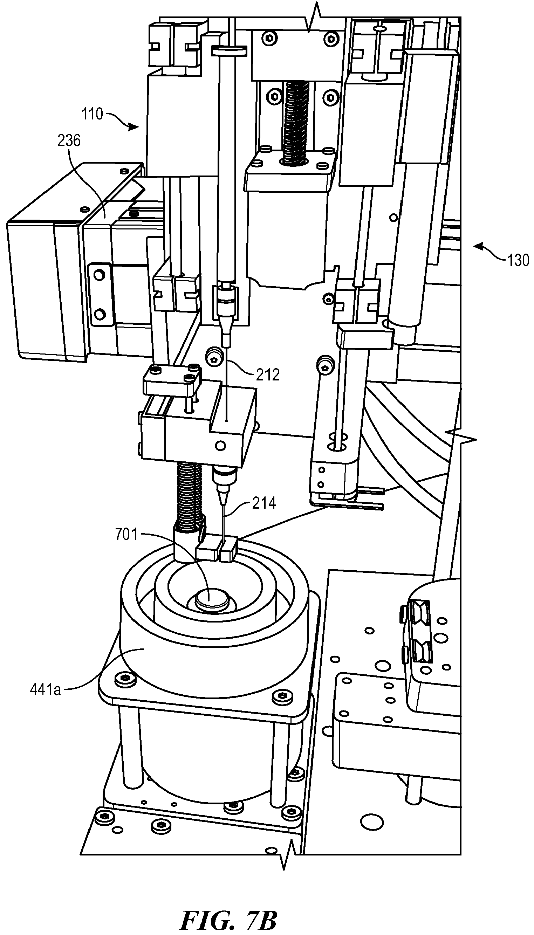

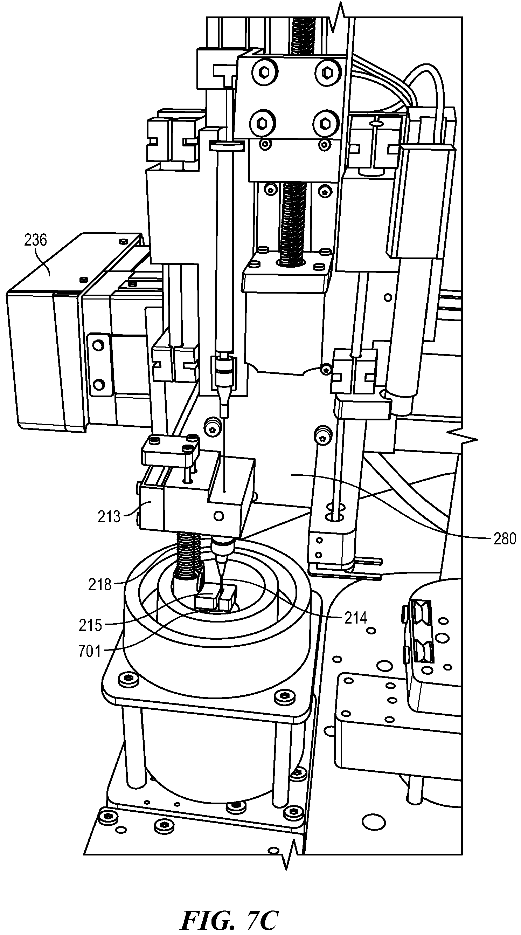

FIG. 7A illustrates an example starting state for the system 100, in which the first pig 441a is holding a source container 701, but the needle assembly 110 is not aligned with the first pig 441a. Accordingly, the system 100 can translate the needle assembly 110 along the second arm 236 of the support assembly 130 until the first needle 212 and the second needle 214 of the needle assembly 110 are vertically aligned with the first pig 441a and the source container 701, as shown in FIG. 7B. Next, with the needle assembly 110 vertically aligned with the source container 701, the needle assembly 110 can be lowered until the needle guide 215 contacts the top of the source container 701, as shown in FIG. 7C. More specifically, the entire support plate 280 can be lowered by lowering the second arm 236 of the support assembly 130 along the elongated slots 139b of the first arm 231 (not visible in FIG. 7C), as described above with reference to FIG. 2A. After the needle guide 215 contacts the top of the source container 701, the support plate 280 is lowered slightly further. As described above with reference to FIG. 2D, this (a) compresses the compression springs 218 and reduces a distance between the needle guide 215 and the needle housing 213, and (b) causes the second needle 214 to protrude distally through the needle guide 215 and into the source container 701.

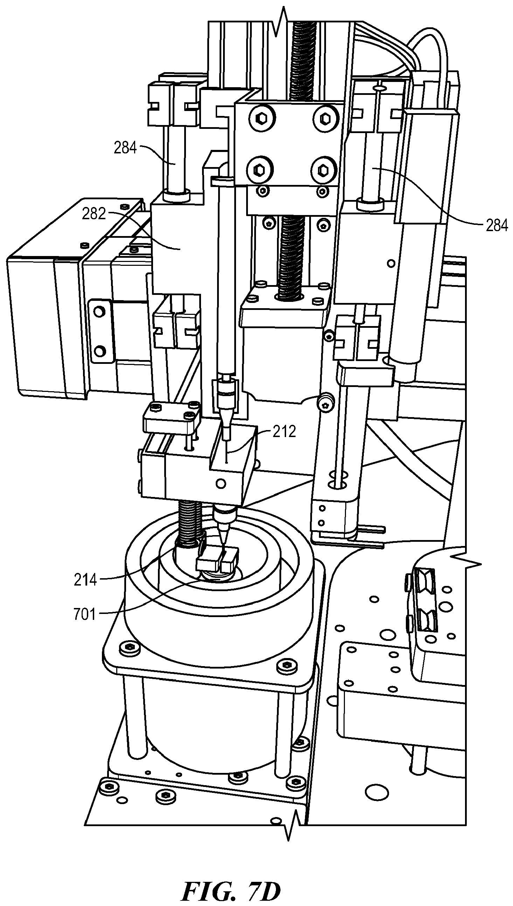

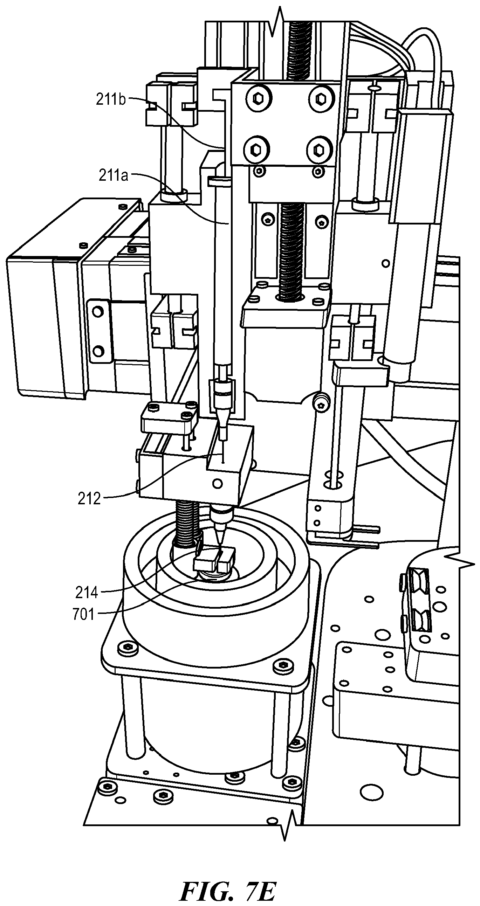

With the second needle 214 positioned within the source container, the first needle 212 can be advanced through the second needle 214 until a distal end of the first needle 212 also enters the source container 701, as shown in FIG. 7D. This can be done by lowering the syringe support frame 282 along the shafts 284 using the support frame motor 283 (not visible in FIG. 7D), as described above with reference to FIG. 2B. Once the first needle 212 is positioned within the source container 701, the system 100 can withdraw the syringe plunger 211b from the syringe barrel 211a, as shown in FIG. 7E. This can be done via the syringe actuator 286 (not visible in FIG. 7D), as described with reference to FIG. 2B. Withdrawing the syringe plunger 211b from the syringe barrel 211a creates a suction force within the first needle 212, which therefore draws up some of the radioactive solution from the source container 701 into the first needle 212. Accordingly, the system 100 can control the distance the syringe plunger 211b is withdrawn to correspond to a desired volume of radioactive solution to withdraw from the source container 701.

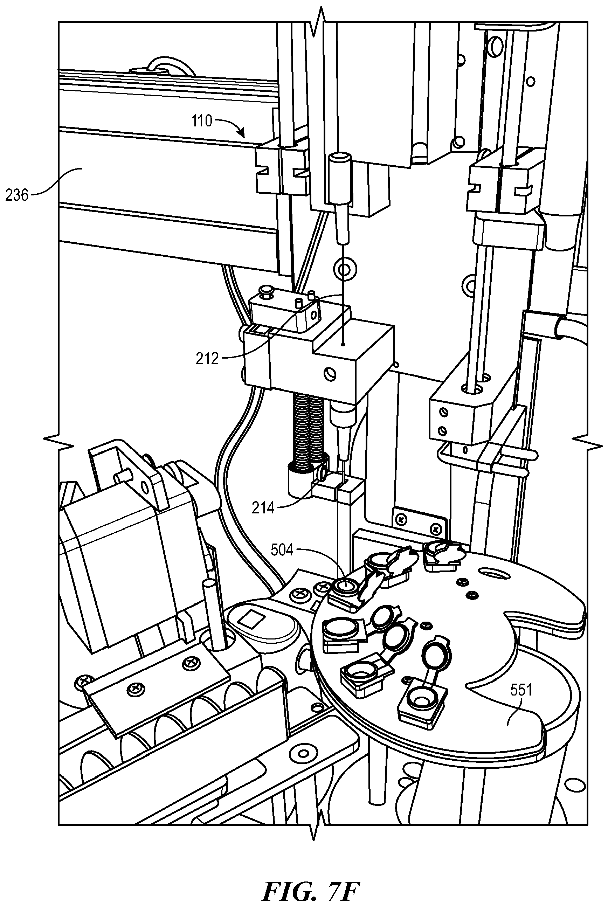

After the radioactive solution is contained within the first needle 212, the first needle 212 is withdrawn from the source container 701 (e.g., by controlling the support frame motor 283 to raise the syringe support frame 282 in a reverse of the operation shown in FIGS. 7C and 7D). The second needle 214 can also be withdrawn from the source container 701 (e.g., by controlling the support assembly 730 to raise the second arm 236 and thus raise the support plate 280 and the needle assembly 110 in a reverse of the operation shown in FIGS. 7B and 7C. With the needle assembly 110 free from the source container 701, the system 100 can move the needle assembly 110 along the second arm 236 (Figure 2A) until the needle assembly 110 aligns with a target transport container 504 positioned within the carousel 551, as shown in FIG. 7F.

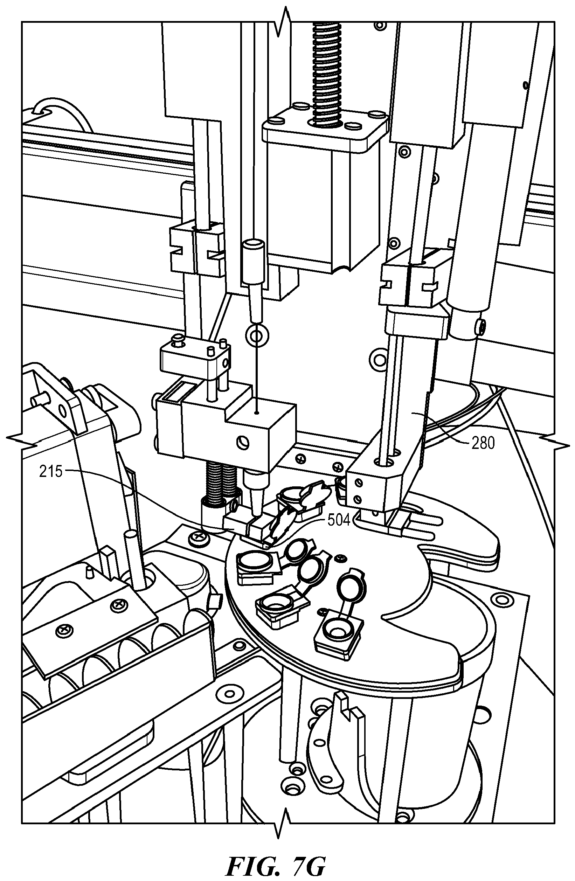

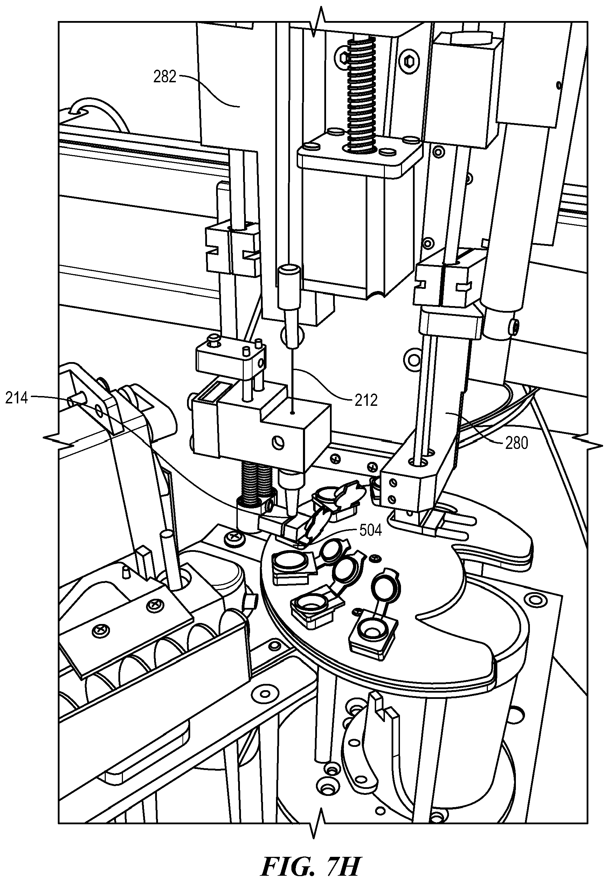

Next, the system 100 can transfer the radioactive solution carried within the first needle 212 to a capsule positioned within the transfer container 504. The system 100 can do so by (a) lowering the support plate 280 using the support assembly 130 until the needle guide 215 is positioned in contact with the transfer container 504, as shown in FIG. 7G, (b) lowering the syringe support frame 282 relative to the support plate 280 using the support frame motor 283 to cause the first needle 212 to advance at least partially through the second needle 214 toward the transfer container 504, as shown in FIG. 7H, and (c) lowering the syringe plunger 211b relative to the syringe barrel 211a to eject the radioactive solution from the first needle 212 and into the capsule positioned within the transport container 504. The operation (b) above can include advancing the first needle 212 until the first needle 212 enters the capsule shell (e.g., the inner capsule in a dual-capsule configuration) before the operation (c) of ejecting the dose from the first needle 212 occurs. The system 100 can then reverse operations (a) and (b) to withdraw the needle assembly 110.

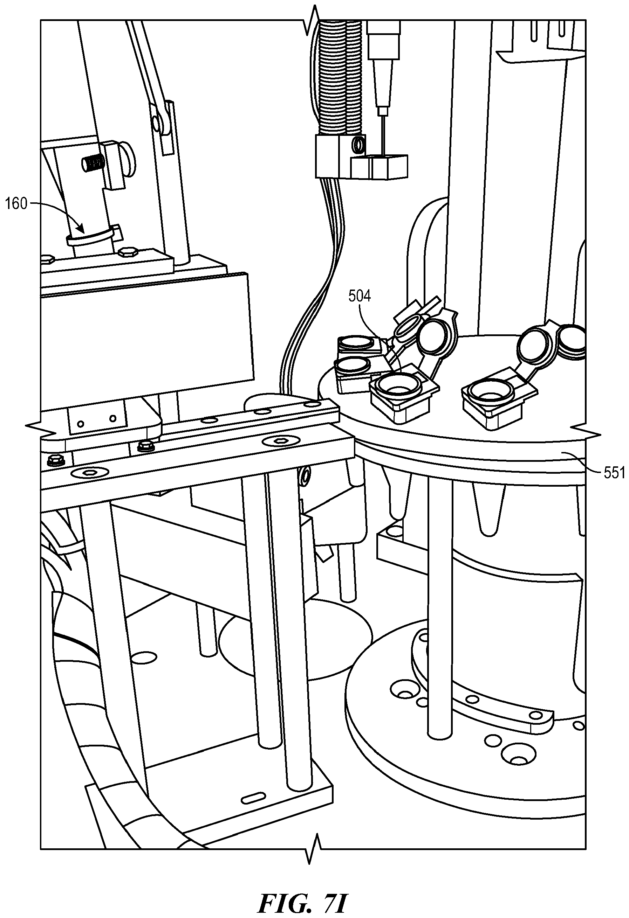

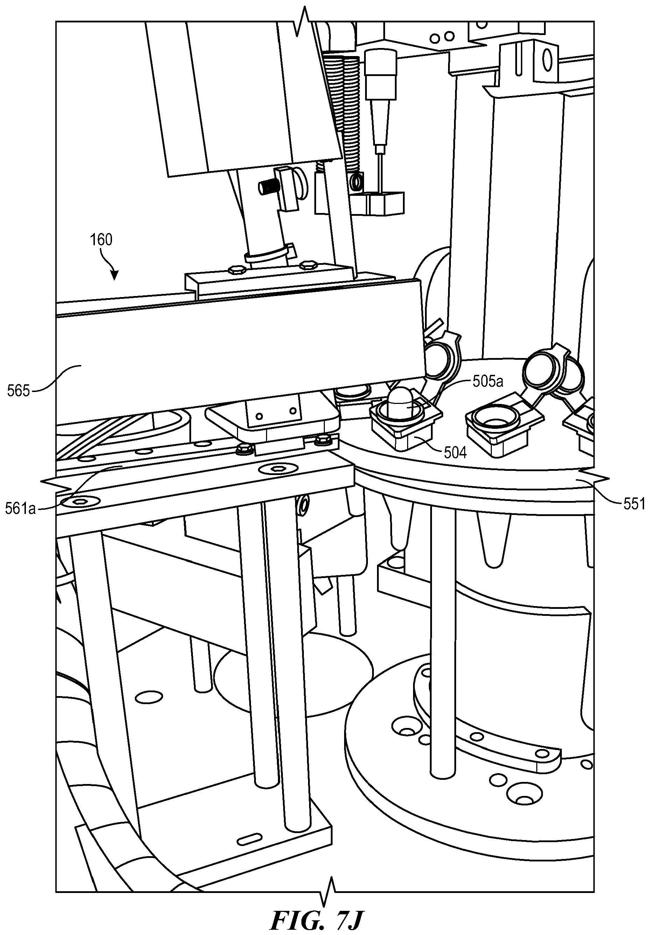

After the capsule in the transport container 504 receives the radioactive dose, the capsule can be capped. To do so, the system 100 can rotate the carousel 551 (e.g., by operating the carousel motor 557 described with reference to FIG. 5C) until the transport container 504 that contains the capsule aligns with the capsule capping assembly 160, as shown in FIG. 7I. Next, the system 100 can advance the spiral pusher frame 565 (and thus the spiral pusher 564) toward the carousel 551 along the rail bracket 561a (e.g., by operating the linear actuator 568 described with reference to FIGS. 5A and 5C) until a distal end of the spiral pusher frame 565 (and thus the spiral pusher 564) aligns with the transport container 504, as shown in FIG. 7J. The spiral pusher motor 566 (FIGS. 5A and 5C) then rotates the spiral pusher 564 (not visible in FIG. 7J) to cause a capsule upper half portion 505a to advance past the distal end of the spiral pusher 564 and the spiral pusher frame 565, as described with reference to FIGS. 5A and 5C. As shown in FIG. 7J, because the spiral pusher 564 is aligned with the transport container 504, the capsule upper half portion 505a falls into the transport container 504 and is generally positioned over the capsule lower half (not visible in FIG. 7J).

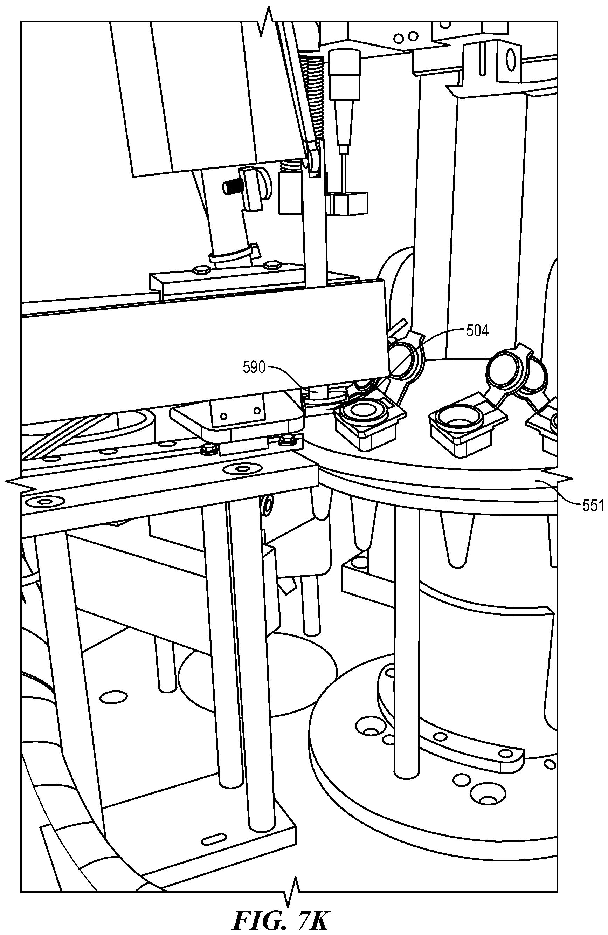

To firmly couple the capsule upper half portion 505a to the capsule lower half, the system 100 rotates the carousel 551 (e.g., by operating the carousel motor 557 described with reference to FIG. 5C) until the transport container 504 aligns with the push rod 590 of the capsule capping assembly 160. When the transport container 504 is aligned with the push rod 590, the system 100 can operate the push rod motor 591 (FIGS. 5A and 5C) to lower the push rod 590 to contact the capsule upper half portion 505a and impart a downward force thereupon, as shown in FIG. 7K. This downward force can firmly couple the capsule upper half portion 505a to the capsule lower half portion 505a (FIG. 5C). In some embodiments, the push rod motor 591 can move the push rod 590 "up and down" several times to ensure a firm coupling between the capsule upper half portion 505a and the capsule lower half portion 505b.

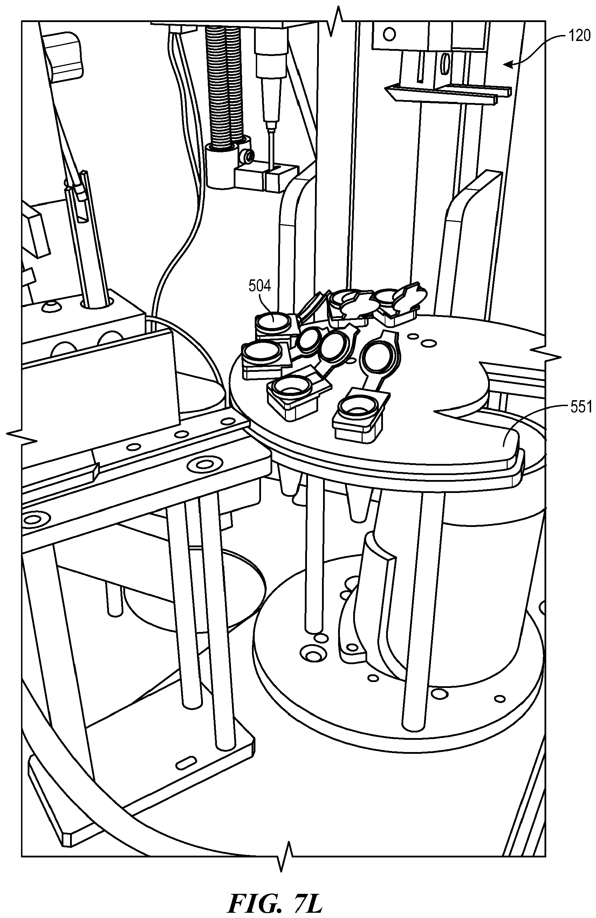

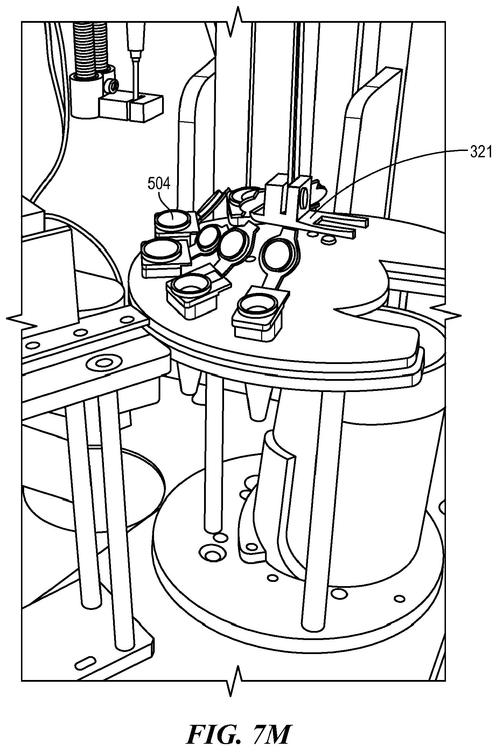

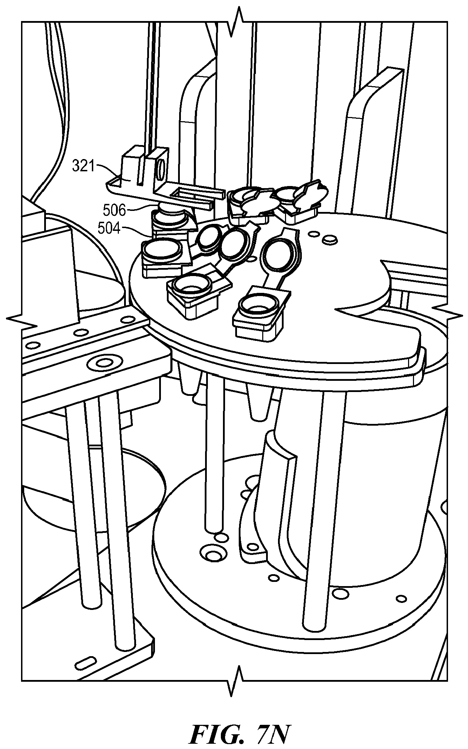

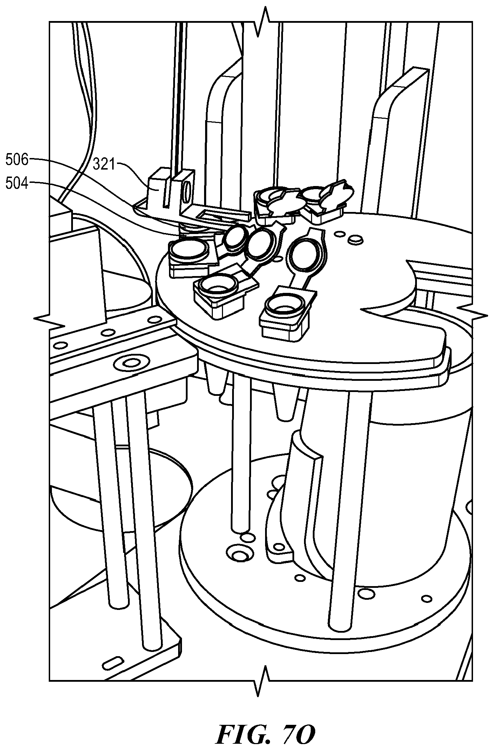

Next, the system 100 can close the transport container 504. To do so, the system 100 rotates the carousel 551 (e.g., by operating the carousel motor 557 described with reference to FIG. 5C) until the transport container 504 aligns with the vial transport assembly 120, as shown in FIG. 7L. The system 100 then lowers the gripper 321 of the vial transport assembly 120 to be proximate the transport container 504 as shown in FIG. 7M. The system 100 can do this by (a) lowering the second arm 236 of the support assembly 130 and thereby lowering the support plate 280 and thus the entire vial transport assembly 120, as described with reference to FIG. 2A, and/or (b) operating the gripper actuator 322 to extend the nesting components 323, as described with reference to FIG. 3. Next, the system 100 translates the gripper 321 in the x-direction (e.g., along the second arm 236 shown in FIG. 2A) until the gripper 321 contacts, and thus partly closes, a cap 506 of the transport container 504, as shown in FIG. 7N. To fully close the cap 506 of the transport container 504, the system 100 can translate the gripper 321 "up and down" in the y-direction one or more times, as shown in FIG. 7O. As set forth above, the system 100 can translate the gripper 321 in the y-direction by (a) lowering the second arm 236 of the support assembly 130 and thereby lowering the support plate 280 and thus the entire vial transport assembly 120, as described with reference to FIG. 2A, and/or (b) operating the gripper actuator 322 to extend the nesting components 323, as described with reference to FIG. 3.

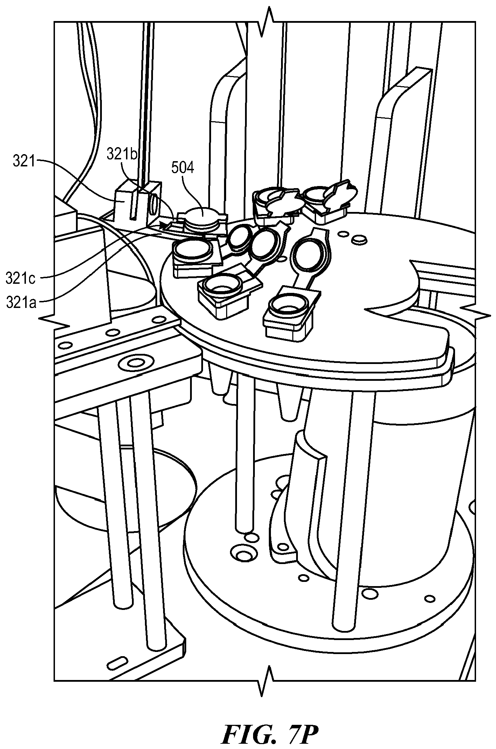

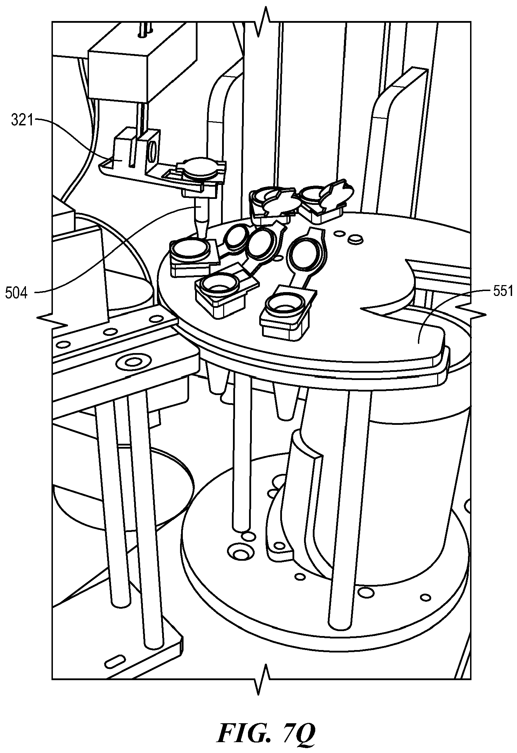

With the cap 506 of the transport container 504 closed, the system 100 can then control the gripper 321 to pick up the transport container 504. For example, as shown in FIG. 7P, the system 100 can move the gripper 321 until the gap 321c between the first prong 321a and the second prong 321b of the gripper 321 aligns with the transport container 504. The system 100 can then move the gripper 321 toward the transport container 504 until the transport container 504 is positioned within the gap 321c between the first prong 321a and the second prong 321b. As shown in FIG. 7Q, the system 100 can then raise the gripper 321 to remove the transport container 504 from the carousel 551.

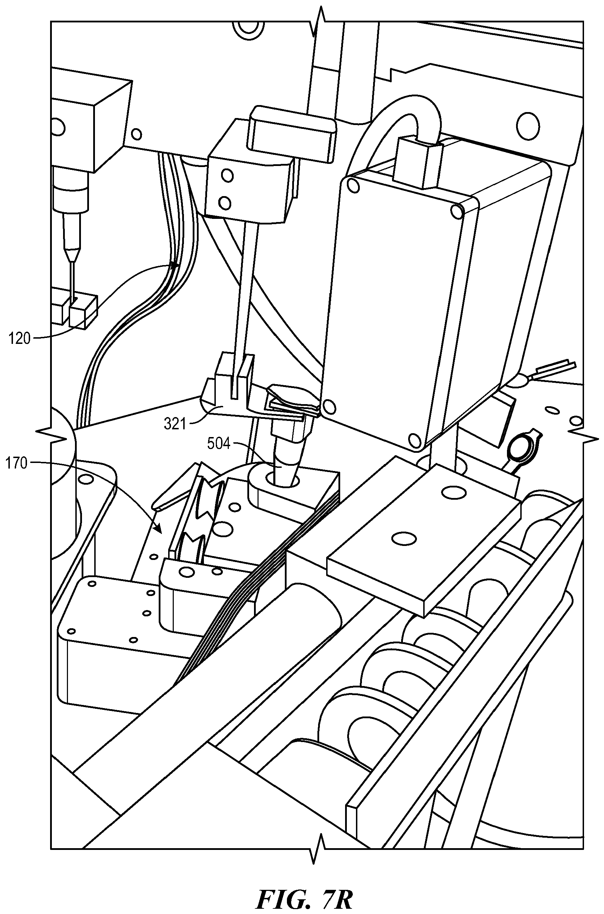

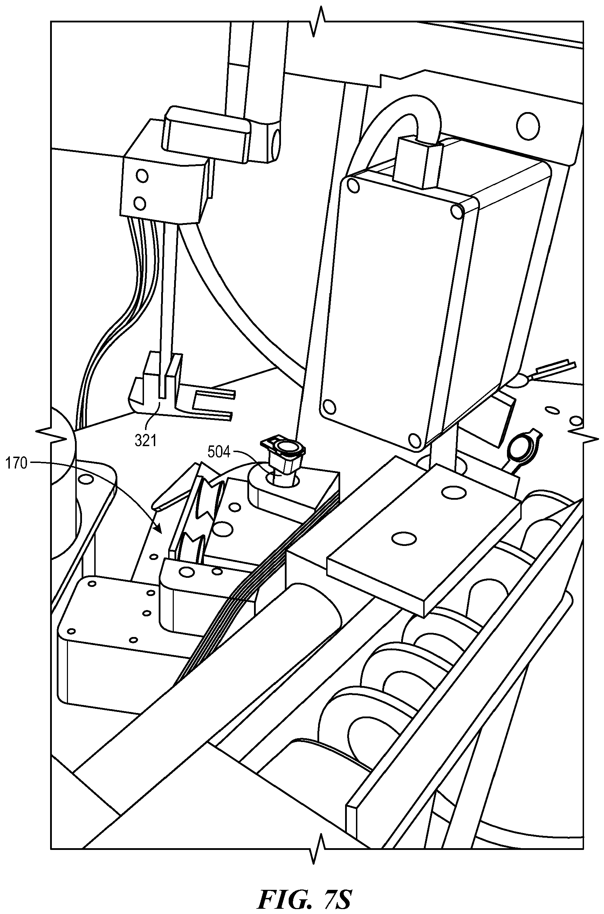

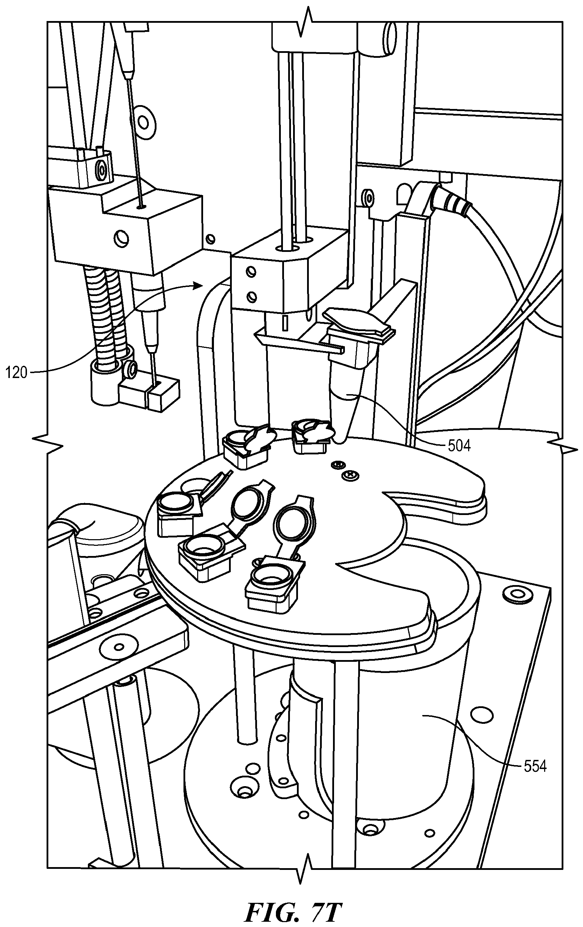

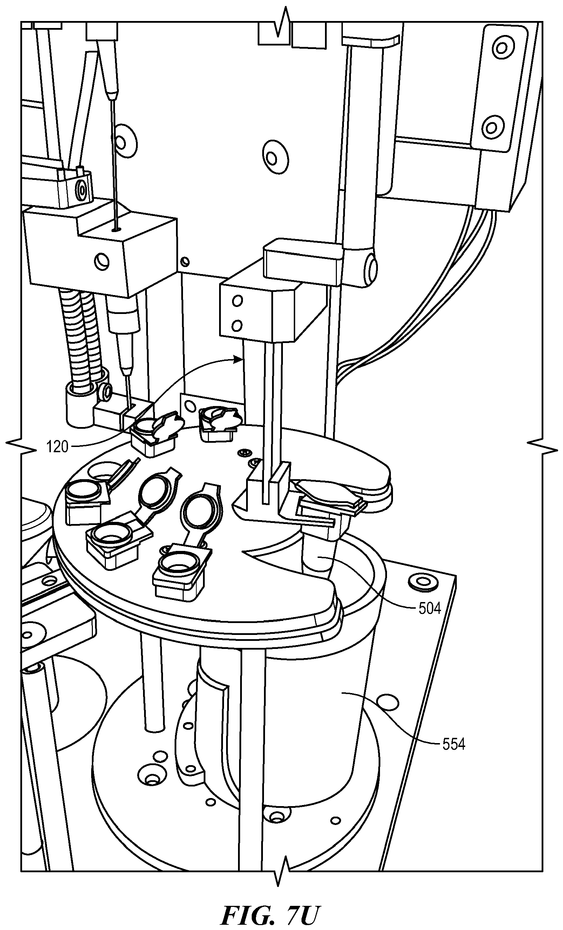

The vial transport assembly 120 then moves the transport container 504 to the dose calibration assembly 170, as shown in FIGS. 7R and 7S. The dose calibration assembly 170 measures the radioactivity of the loaded capsule contained within the transport container 504, as described above with reference to FIGS. 6A-6C. After the radioactivity has been measured, the vial transport assembly once again picks up the transport container 504 and delivers it to the capsule receptacle 554, as shown in FIGS. 7T and 7U. The operations described with respect to FIGS. 7A-7U can then be repeated to load and calibrate another capsule with a radioactive dose.

As one skilled in the art will appreciate, some or all of the operations described above with reference to FIGS. 7A-7U can be automated or semi-automated. For example, in some embodiments a user may set a desired dosage using the user interface 108 shown in FIG. 1 and toggle a "start" button or switch, after which the system 100 automatically performs, without user intervention, the sequence of operations described with reference to FIGS. 7A-7U. In this way, the system 100 is not only expected to reduce user exposure to radioactive material by being remotely operable, but further is expected to increase the efficiency of filling radioactive capsules by minimizing human involvement. Of course, the system 100 may provide further advantages beyond those expressly recited herein.

Moreover, the systems and methods described herein can be used to transport a variety of different radioactive materials. For example, the systems can be used to prepare capsules with iodine-131 ("I-131"), thallium-201 ("Tl-201"), indium-111 ("In-111"), iodine-123 ("I-123"), lutetium-177 ("Lu-177"), copper-64 ("Cu-64"), technetium-99m ("Tc99m"), and/or other radioactive isotopes. The systems and methods can also be used to fill capsules with other compositions, including non-radioactive compositions.

As one skilled in the art will appreciate from the disclosure herein, the motors and actuators described with respect the system 100 can include any motor or actuator suitable for use in an automated filling system. Representative examples include servo motors (including both rotary and linear actuators), linear motors, spindle motors, stepper motors, or the like. Additional representative examples of motors include, but are not limited to, electromagnetic motors, mechanical motors, MEMS motors, micro brushless motors, piezoelectric motors, or the like.

As set forth above, the system 100 of FIGS. 1-7U uses transport containers (e.g., the transport container 504) for moving capsules filled with radioactive material. FIG. 8 is an enlarged view of a representative capsule transport container 800 ("the container 800") configured in accordance with embodiments of the present technology and that can be used with the system 100. As shown, the container 800 includes a body 802 and a cap 804. The body 802 forms a chamber 803 for receiving a capsule (not shown). The cap 802 can reversibly close the chamber 803 via a snap-fit or other connection with the body 804.

In some embodiments, the body 802 can have a capsule retaining feature (not shown) for holding the capsule in a predetermined orientation, e.g., to ensure the capsule is precisely aligned with the needle assembly 110 (FIG. 1) during the capsule filling process. The capsule retaining feature can include on or more flanges, fins, barriers, and/or openings, which can be integral with an inner surface of the body 802 and/or included on an insert or liner that can be placed within the chamber 803. In other embodiments, the interior of the body 802 can simply have a dimension (e.g., diameter) that forces the capsule to have a predetermined orientation when the capsule is placed within the body 802. The body 802 can be sized and shaped to fit within the slots 553 of the carousel 551 (FIG. 5A). The container 800 can be composed of plastic, glass, or another suitable material.

The container 800 further includes a gripper engagement region 808. In operation, the gripper 321 of the vial transport assembly 120 (FIG. 3) can releasably engage the container 800 at the gripper engagement region 808. Accordingly, the gripper engagement region 8008 can be sized and shaped to correspond to the gap 321c formed within the gripper 321 (FIG. 3). For example, in embodiments in which the gap 321c has a generally rectangular shape, the gripper engagement region 808 can have a generally rectangular or "D"-shaped cross-sectional shape. As another example, in embodiments in which the gap 321c has a curved shape, the gripper engagement region 808 can also have a curved or rounded cross-sectional shape. Regardless of the shape of the gripper engagement region 808, the gripper engagement region 8008 can include a flange 809 that can be engaged by the prongs 321a, 321b of the gripper 321 (FIG. 3) to assist with grabbing and carrying the container 800.

### Conclusion

Certain aspects of the systems and methods described herein can be implemented with and/or distributed across computing architecture. For example, many of the systems described herein include a memory storing data, software modules, instructions, or the like. The memories described herein can include one or more of various hardware devices for volatile and non-volatile storage, and can include both read-only and writable memory. For example, a memory can comprise random access memory (RAM), various caches, CPU registers, read-only memory (ROM), and writable non-volatile memory, such as flash memory, hard drives, floppy disks, CDs, DVDs, magnetic storage devices, tape drives, device buffers, and so forth. A memory is not a propagating signal divorced from underlying hardware; a memory is thus non-transitory. In some embodiments, the memory is a non-transitory computer-readable storage medium that stores, for example, programs, software, data, or the like.

As one of skill in the art will appreciate from the disclosure herein, various components of the systems described above can be omitted without deviating from the scope of the present technology. Likewise, additional components not explicitly described above may be added to the systems without deviating from the scope of the present technology. For example, it will be appreciated that specific embodiments of the technology have been described herein for purposes of illustration, but well-known structures and functions have not been shown or described in detail to avoid unnecessarily obscuring the description of the embodiments of the technology. Moreover, although specific embodiments of, and examples for, the technology are described above for illustrative purposes, various equivalent modifications are possible within the scope of the technology as those skilled in the relevant art will recognize. For example, although steps are presented in a given order, alternative embodiments may perform steps in a different order. The various embodiments described herein may also be combined to provide further embodiments. Accordingly, the present technology is not limited to the configurations expressly identified herein, but rather encompasses variations and alterations of the described systems and methods.

Further, while advantages associated with some embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

Unless the context clearly requires otherwise, throughout the description and the examples, the words "comprise," "comprising," and the like are to be construed in an inclusive sense, as opposed to an exclusive or exhaustive sense; that is to say, in the sense of "including, but not limited to." As used herein, the terms "connected," "coupled," or any variant thereof, means any connection or coupling, either direct or indirect, between two or more elements; the coupling of connection between the elements can be physical, logical, or a combination thereof. Additionally, the words "herein," "above," "below," and words of similar import, when used in this application, shall refer to this application as a whole and not to any particular portions of this application. Where the context permits, words in the above Detailed Description using the singular or plural number may also include the plural or singular number respectively. As used herein, the phrase "and/or" as in "A and/or B" refers to A alone, B alone, and A and B. Further, where specific integers are mentioned herein which have known equivalents in the art to which the embodiments relate, such known equivalents are deemed to be incorporated herein as if individually set forth.

Aspects of the invention are described below with reference to the following numbered clauses:
1. A capsule filling system for transferring a dose of a radioactive material from a source container into a capsule, the capsule filling system comprising:
   a holder assembly having:
      a rotatable carousel with one or more openings sized and shaped to releasably hold one or more corresponding transport containers, and
      a capsule receptacle;
   a needle assembly having a syringe with a needle;
   a transport assembly having a moveable gripper sized and shaped to releasably grasp one of the one or more transport containers; and
   a controller configured to remotely control operation of the capsule filling system to (a) cause the needle assembly to withdraw a dose of the radioactive material from the source container, (b) control the needle assembly to transfer the dose to an empty capsule positioned in one of the one or more transport containers in the holder assembly, and (c) control the transport assembly to transfer the transport container with the filled capsule to the capsule receptacle.
2. The capsule filling system of clause 1, further comprising a moveable support assembly, the moveable support assembly having:
   a first support arm extending generally vertically;
   a second support arm extending generally horizontally, wherein the second support arm is translationally coupled to the first support arm to move along the first support arm in a vertical direction; and
   a support plate translationally coupled to the second support arm to move along the second support arm in a horizontal direction,
   wherein the needle assembly and the transport assembly are coupled to the support plate.
3. The capsule filling system of clause 2 wherein the controller is configured to move the second support arm relative to the first support arm and/or the support plate relative to the second support arm to perform the operations (a), (b), and (c).
4. The capsule filling system of any preceding clause, further comprising a pig assembly having at least one pig for holding the source container.
5. The capsule filling system of clause 4 wherein the pig assembly includes at least two pigs for holding two different source containers, and wherein the at least two pigs are translationally coupled to a rail system, and wherein the controller is configured to selectively translate the at least two pigs along the rail system and relative to the needle assembly to align a select one of the at least two pigs with the needle assembly.
6. The capsule filling system of any preceding clause wherein:
   the needle is a first needle,
   the needle assembly further comprises a second needle, and
   the first needle is sized and shaped to extend concentrically within the second needle to withdraw the dose from the source container.
7. The capsule filling system of any preceding clause, further comprising a capsule capping assembly, the capsule capping assembly comprising:
   a shell delivery mechanism for transporting first portions of capsule shells toward the holder assembly; and
   a push rod coupled to a push rod motor via one or more articulating arms,
   wherein the controller is further configured to (a) rotate the carousel to align a particular transport container holding a second portion of the capsule shell with the capsule capping assembly, (b) actuate the shell delivery mechanism to deliver the first portion of the capsule shell to the transport container, and (c) control the push rod motor to depress the push rod into the transport container via movement of the one or more articulating arms to secure the first portion of the capsule shell to the second portion of the capsule shell.
8. The capsule filling system of any preceding clause, further comprising a dose calibration assembly having a dose calibrator for measuring a radioactivity of the filled capsule, wherein the controller is configured to cause the transport assembly to transfer the transport container with the filled capsule to the dose calibration assembly to measure the radioactivity of the filled capsule before transferring the transport container to the capsule receptacle.
9. The capsule filling system of any preceding clause, further comprising a housing enclosing the holder assembly, the needle assembly, and the transport assembly.
10. The capsule filling system of any preceding clause wherein the controller includes a computing system including:
   a user interface for receiving a user input;
   a non-transitory computer readable memory storing instructions for controlling the operations (a), (b), and (c); and
   a processor that executes the instructions in response to the user input to execute the operations (a), (b), and (c).
11. A capsule filling system for transferring a dose of a radioactive material from a source container into a capsule, the capsule filling system comprising:
   a support plate;
   a needle assembly coupled to the support plate and configured to remove a dose of the radioactive material from the source container, the needle assembly including:
      a syringe support frame translationally coupled to the support plate,
      a syringe having a syringe barrel, a syringe plunger, and a first needle, wherein the syringe barrel is translationally fixed to the syringe support frame,
      a syringe actuator translatable relative to the syringe support frame and translationally fixed to the syringe plunger to move the syringe plunger relative to the syringe barrel,
      a needle housing fixed to the support frame and having a passage extending therethrough sized and shaped to slidably receive the first needle, and
      a second needle extending from the needle housing and aligned with the needle passage, wherein the second needle has an inner diameter that is greater than an outer diameter of the first needle; and
   a controller configured to (a) translate the support plate toward the source container until the second needle is in contact with the source container, (b) with the second needle in contact with the source container, translate the syringe support frame toward the source container and relative to the support plate to advance the first needle through the needle passage and the second needle and into fluidic contact with the radioactive material, and (c) actuate the syringe actuator to translate the syringe plunger relative to the syringe barrel to draw the dose of the radioactive material into the first needle and/or the syringe barrel.
12. The capsule filling system of clause 11 wherein:
   the needle assembly further comprises a needle guide coupled to the needle housing by a compression spring and having a slot sized and shaped to receive a distal tip portion of the second needle, and
   in response to the controller causing the support plate to translate toward the source container, the needle guide contacts the source container and at least partially compresses the compression spring to permit the distal tip portion of the second needle to extend past the needle guide and enter the source container.
13. The capsule filling system of any of clauses 11-12 wherein the syringe support frame is translationally coupled to the support plate via one or more vertical shafts, and wherein the needle assembly further includes a support frame motor for translationally moving the syringe support frame along the one or more vertical shafts.
14. The capsule filling system of any of clauses 11-13 wherein the needle assembly further includes an optical sensor configured to track a position of the first needle.
15. The capsule filling system of clause 14 wherein the controller is configured to control operation of the needle assembly based at least in part on feedback from the optical sensor.
16. The capsule filling system of any of clauses 11-15 wherein a ratio of the inner diameter of the second needle to the outer diameter of the first needle is between about 5:1 and about 1.2:1.
17. The capsule filling system of any of clauses 11-16 wherein the first needle and the second needle form an annular channel when the first needle advances concentrically within the second needle, and wherein the needle assembly further comprises a filter fluidly coupled to the annular channel for capturing radioactive particles traveling through the annular channel.
18. The capsule filling system of clause 17 wherein the housing includes a passage that extends between the annular channel and the filter, and wherein the passage includes a material configured to capture and retain the radioactive particles.
19. The capsule filling system of any of clauses 11-18, further comprising a pig assembly configured to hold the source container of the radioactive material.
20. The capsule filling system of clause 19 wherein the pig assembly includes a first pig configured to hold a first source container and a second pig configured to hold a second source container, and wherein the first pig and the second pig are translatable relative to the needle assembly to selectively align either the first source container or the second source container with the needle assembly.
21. The capsule filling system of clause 20 wherein the pig assembly further includes a pig support plate and a pig positioning plate, and wherein the first pig and the second pig are positioned on the pig support plate in predetermined positions defined by the pig positioning plate.
22. The capsule filling system of clause 21 wherein the pig support plate is slidably coupled to a rail system for moving the first pig and/or the second pig relative to the needle assembly.
23. A capsule filling system, the capsule filling system comprising:
   a base plate;
   a holder assembly operably coupled to the base plate, the holder assembly including a carousel having a plurality of openings for releasably holding a corresponding plurality of transport containers, wherein the carousel is rotatable relative to the base plate;
   a capsule capping assembly operably coupled to the base plate, the capsule capping assembly including:
      a shell delivery mechanism for transporting first portions of capsule shells toward the carousel, and
      a pusher; and
   a controller configured to (a) rotate the carousel to align a particular opening holding a particular transport container having a second portion of a particular capsule shell with the capsule capping assembly, (b) actuate the shell delivery mechanism to deliver the first portion of the particular capsule shell to the transport container, and (c) control the pusher to secure the first portion of the particular capsule shell to the second portion of the particular capsule shell.
24. The capsule filling system of clause 23 wherein:
   the carousel includes a first plate spaced apart from a second plate by one or more elongated spacers,
   the first plate includes the plurality of slots for releasably holding the plurality of transport containers, and
   the holder assembly further includes a capsule receptacle positioned on the second plate and at least partially occupying a space between the first plate and the second plate.
25. The capsule filling system of clause 24 wherein the holder assembly further includes a positioner coupled to the second plate of the carousel, the positioner configured to control a position of the capsule receptacle.
26. The capsule filling system of any of clauses 23-25 wherein the shell delivery mechanism is translationally coupled to a mounting plate that is fixedly coupled to the base plate such that the shell delivery mechanism can translate relative to the carousel to position the shell delivery mechanism over the carousel.
27. The capsule filling system of any of clauses 23-26 wherein the shell delivery mechanism includes a spiral pusher positioned within a frame.
28. The capsule filling system of any of clauses 23-27 wherein the shell delivery mechanism includes a conveyor belt.
29. The capsule filling system of any of clauses 23-28, further comprising a transport assembly, wherein the controller is further configured to (a) control the transport assembly to close the transport container after the pusher secures the first portion of the particular shell to the second portion of the particular shell, and (b) move the transport container to a capsule receptacle.

## Claims

1. A capsule filling system for transferring a dose of a radioactive material from a source container into a capsule, the capsule filling system comprising:
a holder assembly having:
a rotatable carousel with one or more openings sized and shaped to releasably hold one or more corresponding transport containers, and
a capsule receptacle;
a needle assembly having a syringe with a needle;
a transport assembly having a moveable gripper sized and shaped to releasably grasp one of the one or more transport containers; and
a controller configured to remotely control operation of the capsule filling system to (a) cause the needle assembly to withdraw a dose of the radioactive material from the source container, (b) control the needle assembly to transfer the dose to an empty capsule positioned in one of the one or more transport containers in the holder assembly, and (c) control the transport assembly to transfer the transport container with the filled capsule to the capsule receptacle.

2. The capsule filling system of claim 1, further comprising a moveable support assembly, the moveable support assembly having:
a first support arm extending generally vertically;
a second support arm extending generally horizontally, wherein the second support arm is translationally coupled to the first support arm to move along the first support arm in a vertical direction; and
a support plate translationally coupled to the second support arm to move along the second support arm in a horizontal direction,
wherein the needle assembly and the transport assembly are coupled to the support plate, preferably wherein the controller is configured to move the second support arm relative to the first support arm and/or the support plate relative to the second support arm to perform the operations (a), (b), and (c).

3. The capsule filling system of any preceding claim, further comprising a pig assembly having at least one pig for holding the source container, preferably wherein the pig assembly includes at least two pigs for holding two different source containers, and wherein the at least two pigs are translationally coupled to a rail system, and wherein the controller is configured to selectively translate the at least two pigs along the rail system and relative to the needle assembly to align a select one of the at least two pigs with the needle assembly.

4. The capsule filling system of any preceding claim wherein:
the needle is a first needle,
the needle assembly further comprises a second needle, and
the first needle is sized and shaped to extend concentrically within the second needle to withdraw the dose from the source container.

5. The capsule filling system of any preceding claim, further comprising a capsule capping assembly, the capsule capping assembly comprising:
a shell delivery mechanism for transporting first portions of capsule shells toward the holder assembly; and
a push rod coupled to a push rod motor via one or more articulating arms,
wherein the controller is further configured to (a) rotate the carousel to align a particular transport container holding a second portion of the capsule shell with the capsule capping assembly, (b) actuate the shell delivery mechanism to deliver the first portion of the capsule shell to the transport container, and (c) control the push rod motor to depress the push rod into the transport container via movement of the one or more articulating arms to secure the first portion of the capsule shell to the second portion of the capsule shell; and/or further comprising a dose calibration assembly having a dose calibrator for measuring a radioactivity of the filled capsule, wherein the controller is configured to cause the transport assembly to transfer the transport container with the filled capsule to the dose calibration assembly to measure the radioactivity of the filled capsule before transferring the transport container to the capsule receptacle; and/or further comprising a housing enclosing the holder assembly, the needle assembly, and the transport assembly; and/or wherein the controller includes a computing system including:
a user interface for receiving a user input;
a non-transitory computer readable memory storing instructions for controlling the operations (a), (b), and (c); and
a processor that executes the instructions in response to the user input to execute the operations (a), (b), and (c).

6. A capsule filling system for transferring a dose of a radioactive material from a source container into a capsule, the capsule filling system comprising:
a support plate;
a needle assembly coupled to the support plate and configured to remove a dose of the radioactive material from the source container, the needle assembly including:
a syringe support frame translationally coupled to the support plate,
a syringe having a syringe barrel, a syringe plunger, and a first needle, wherein the syringe barrel is translationally fixed to the syringe support frame,
a syringe actuator translatable relative to the syringe support frame and translationally fixed to the syringe plunger to move the syringe plunger relative to the syringe barrel,
a needle housing fixed to the support frame and having a passage extending therethrough sized and shaped to slidably receive the first needle, and
a second needle extending from the needle housing and aligned with the needle passage, wherein the second needle has an inner diameter that is greater than an outer diameter of the first needle; and
a controller configured to (a) translate the support plate toward the source container until the second needle is in contact with the source container, (b) with the second needle in contact with the source container, translate the syringe support frame toward the source container and relative to the support plate to advance the first needle through the needle passage and the second needle and into fluidic contact with the radioactive material, and (c) actuate the syringe actuator to translate the syringe plunger relative to the syringe barrel to draw the dose of the radioactive material into the first needle and/or the syringe barrel.

7. The capsule filling system of claim 6 wherein:
the needle assembly further comprises a needle guide coupled to the needle housing by a compression spring and having a slot sized and shaped to receive a distal tip portion of the second needle, and
in response to the controller causing the support plate to translate toward the source container, the needle guide contacts the source container and at least partially compresses the compression spring to permit the distal tip portion of the second needle to extend past the needle guide and enter the source container.

8. The capsule filling system of any of claims 6-7 wherein the syringe support frame is translationally coupled to the support plate via one or more vertical shafts, and wherein the needle assembly further includes a support frame motor for translationally moving the syringe support frame along the one or more vertical shafts.

9. The capsule filling system of any of claims 6-8 wherein the needle assembly further includes an optical sensor configured to track a position of the first needle, preferably wherein the controller is configured to control operation of the needle assembly based at least in part on feedback from the optical sensor.

10. The capsule filling system of any of claims 6-9 wherein a ratio of the inner diameter of the second needle to the outer diameter of the first needle is between about 5:1 and about 1.2:1; and/or wherein the first needle and the second needle form an annular channel when the first needle advances concentrically within the second needle, and wherein the needle assembly further comprises a filter fluidly coupled to the annular channel for capturing radioactive particles traveling through the annular channel, preferably wherein the housing includes a passage that extends between the annular channel and the filter, and wherein the passage includes a material configured to capture and retain the radioactive particles.

11. The capsule filling system of any of claims 6-10, further comprising a pig assembly configured to hold the source container of the radioactive material, preferably wherein the pig assembly includes a first pig configured to hold a first source container and a second pig configured to hold a second source container, and wherein the first pig and the second pig are translatable relative to the needle assembly to selectively align either the first source container or the second source container with the needle assembly, optionally wherein the pig assembly further includes a pig support plate and a pig positioning plate, and wherein the first pig and the second pig are positioned on the pig support plate in predetermined positions defined by the pig positioning plate, optionally wherein the pig support plate is slidably coupled to a rail system for moving the first pig and/or the second pig relative to the needle assembly.

12. A capsule filling system, the capsule filling system comprising:
a base plate;
a holder assembly operably coupled to the base plate, the holder assembly including a carousel having a plurality of openings for releasably holding a corresponding plurality of transport containers, wherein the carousel is rotatable relative to the base plate;
a capsule capping assembly operably coupled to the base plate, the capsule capping assembly including:
a shell delivery mechanism for transporting first portions of capsule shells toward the carousel, and
a pusher; and
a controller configured to (a) rotate the carousel to align a particular opening holding a particular transport container having a second portion of a particular capsule shell with the capsule capping assembly, (b) actuate the shell delivery mechanism to deliver the first portion of the particular capsule shell to the transport container, and (c) control the pusher to secure the first portion of the particular capsule shell to the second portion of the particular capsule shell.

13. The capsule filling system of claim 12 wherein:
the carousel includes a first plate spaced apart from a second plate by one or more elongated spacers,
the first plate includes the plurality of slots for releasably holding the plurality of transport containers, and
the holder assembly further includes a capsule receptacle positioned on the second plate and at least partially occupying a space between the first plate and the second plate, preferably wherein the holder assembly further includes a positioner coupled to the second plate of the carousel, the positioner configured to control a position of the capsule receptacle.

14. The capsule filling system of any of claims 12-13 wherein the shell delivery mechanism is translationally coupled to a mounting plate that is fixedly coupled to the base plate such that the shell delivery mechanism can translate relative to the carousel to position the shell delivery mechanism over the carousel and/or wherein the shell delivery mechanism includes a spiral pusher positioned within a frame and/or wherein the shell delivery mechanism includes a conveyor belt.

15. The capsule filling system of any of claims 12-14, further comprising a transport assembly, wherein the controller is further configured to (a) control the transport assembly to close the transport container after the pusher secures the first portion of the particular shell to the second portion of the particular shell, and (b) move the transport container to a capsule receptacle.
